(19) **Europäisches Patentamt European Patent Office Office européen des brevets**

(11) **EP 1 727 459 B1**

(12) **EUROPEAN PATENT SPECIFICATION**

(45) Date of publication and mention
of the grant of the patent:
**15.04.2009 Bulletin 2009/16**

(51) Int Cl.:
*A61B 1/005* (2006.01)  *A61M 25/01* (2006.01)

(21) Application number: **05713867.9**

(22) Date of filing: **17.02.2005**

(86) International application number:
**PCT/US2005/005425**

(87) International publication number:
**WO 2005/081202 (01.09.2005 Gazette 2005/35)**

(54) **VARIABLE STEERABLE CATHETERS**

VARIABEL LENKBARE KATHETER

CATHETERS ORIENTABLES ET VARIABLES

(84) Designated Contracting States:
**AT BE BG CH CY CZ DE DK EE ES FI FR GB GR HU IE IS IT LI LT LU MC NL PL PT RO SE SI SK TR**

(30) Priority: **17.02.2004 US 545865 P**
**01.03.2004 US 549343 P**
**01.03.2004 US 549344 P**
**11.02.2005 US 57074**

(43) Date of publication of application:
**06.12.2006 Bulletin 2006/49**

(73) Proprietor: **ACUMEN MEDICAL, INC.**
**Mountain View, CA 94043 (US)**

(72) Inventors:
• **LEEFLANG, Stephen, A.**
**Sunnyvale, CA 94085 (US)**
• **LOFTHOUSE, Trevor, A.**
**Sunnyvale, CA 94086 (US)**

• **MORRISON, George**
**Redwood Shores, CA 94065 (US)**
• **MOURLAS, Nicholas, J.**
**Mountain View, CA 94040 (US)**
• **EVERSULL, Christian, S.**
**Palo Alto, CA 94306 (US)**
• **HOFTEN VAN, Jamie, J.**
**San Francisco, CA 94122 (US)**
• **VENTURA, Christine, P.**
**San Jose, CA 95130 (US)**

(74) Representative: **Viering, Hans-Martin**
**Viering, Jentschura & Partner**
**Postfach 22 14 43**
**80504 München (DE)**

(56) References cited:
**WO-A-01/03766**          **US-A- 5 489 270**
**US-A1- 2001 039 418**

## Description

<u>FIELD OF THE INVENTION</u>

The present invention relates to an apparatus according to the preamble of claim 1. In particular, the present invention relates generally to catheters for introduction into body lumens within a patient's body, and, more particularly, to steerable catheters for visualization within a patient's body and/or for accessing body lumens.

<u>BACKGROUND</u>

An apparatus of the initially-mentioned type is known, e.g., from US-A-5 489 270. Minimally invasive procedures have been implemented in a variety of medical settings, e.g., for vascular interventions, such as angioplasty, stenting, embolic protection, electrical heart stimulation, heart mapping and visualization, tissue ablation, and the like. One such procedure involves delivering an electrical lead into a coronary vein of a patient's heart that may be used to electrically stimulate the heart. Another procedure involves delivering an electrode probe into a patient's heart to ablate tissue, e.g., surrounding the pulmonary ostia to treat atrial fibrillation.

Steerable catheters have also been suggested to facilitate delivering such devices. Such devices generally have a fixed relationship between deflection and radius of curvature and arc length. It has been suggested to provide a stiffening member that may be advanced or retracted within a steerable section of a catheter. Such a stiffening member may allow steering only on a portion of the catheter beyond the stiffening member. However, such stiffening members may also change the stiffness of all or portions of the catheter, which may be undesirable, e.g., when passing the catheter through tortuous anatomy.

During catheter-based procedures, instruments, fluids, and/or medicaments may be delivered within a patient's vasculature using visualization tools, such as x-ray, fluoroscopy, ultrasound imaging, endoscopy, and the like. In many procedures, it may be desirable to deliver instruments through opaque fluids, such as blood, or other materials. Endoscopes have been suggested that include devices for displacing these materials from an optical path, e.g., by introducing a clear fluid from the endoscope in an attempt to clear its field of view. Yet there are still improvements that may be made to such devices.

Accordingly, apparatus and methods for imaging within body lumens and/or for delivering instruments and/or fluids into a patient's body would be useful.

<u>SUMMARY OF THE INVENTION</u>

The present invention provides an apparatus according to claim 1.

Further embodiments of the apparatus according to the present invention are described in the dependent claims.

In accordance with an embodiment, an apparatus is provided that includes a tubular member including a proximal end, a distal end sized for introduction into a body lumen, and a passage extending along a steerable distal portion of the tubular member. The passage includes a first region extending substantially parallel to a center of modulus of the distal portion and a second region offset from the center of modulus. A steering element is disposed through the passage extending along the distal portion that includes a proximal end disposed adjacent to the tubular member proximal end, and a distal end fixed to the tubular member distal end beyond the distal portion.

A steering adjustment member is slidably disposed within the passage for selectively directing a portion of the steering element between the first and second regions. For example, the steering adjustment member may constrain the steering element within the first region proximal to a distal tip of the steering adjustment member and allow the steering element to enter the second region beyond the distal tip of the steering adjustment member. Thus, the steering adjustment member may change a fulcrum initiation location on the distal portion of the tubular member, a bending moment applied to the distal portion when an axial force is applied to the proximal end of the steering element, and/or a radius of curvature of the distal portion.

The distal end of the tubular member may be advanced into a body lumen. An axial force may be applied to the steering element extending from the proximal end of the tubular member to the distal portion of the tubular member to cause the distal portion to curve or bend, and a portion of the steering element may be directed within the distal portion from the first region aligned within a center of modulus of the distal portion to the second region within the distal portion, thereby adjusting a radius of curvature of the distal portion.

In one embodiment, the steering element may be constrained within the first region by a steering adjustment member, and may be directed into the second region beyond the steering adjustment member when an axial force is applied to the steering element. Optionally, the steering adjustment member may be slidable axially within the distal portion to change a fulcrum initiation location from which the distal portion bends.

Other aspects and features of the present invention will become apparent from consideration of the following description taken in conjunction with the accompanying drawings.

<u>BRIEF DESCRIPTION OF THE DRAWINGS</u>

FIG. 1 is a side view of an apparatus, including an imaging catheter having a handle on a proximal end, a balloon on a distal end, a syringe for expanding the balloon, and a monitor for displaying images obtained by the catheter through the balloon.

FIG. 2 is a side view of the catheter of the apparatus of FIG. 1.

FIG. 3 is a side view detail of the distal end of the catheter of FIG. 1, with the balloon in an expanded condition.

FIGS. 4A-4E are cross-sectional views of the catheter of FIG. 2 taken along lines 4A-4A, 4B-4B, 4C-4C, 4D-4D, and 4E-4E, respectively.

FIG. 5 is a side view of the handle of the apparatus of FIG. 1.

FIGS. 6A and 6B are cross-sectional perspective and side views, respectively, of the handle of FIG. 5.

FIG. 7 is a schematic showing components of an imaging assembly that may be included with the apparatus of FIG. 1.

FIGS. 8A and 8B are side views of another embodiment of an apparatus including a needle for delivering one or more agents into tissue.

FIGS. 9A-9C are cross-sectional views of a patient's heart, showing a method for introducing an apparatus into a chamber of the heart to deliver one or more agents into heart tissue.

FIGS. 10A-10D are cross-sectional views of a patient's heart, showing a method for introducing an apparatus into a first chamber of the heart to create a puncture through a wall of the heart into a second chamber of the heart.

FIGS. 11A and 11B are cross-sectional views of an embodiment of an imaging apparatus including a catheter having an expandable sheath that provides an expandable accessory lumen.

FIGS. 12A and 12B are cross-sectional views of another embodiment of an imaging apparatus including a catheter having an expandable sheath that provides an expandable accessory lumen.

FIGS. 13A and 13B are cross-sectional views of still another embodiment of an imaging apparatus including a coiled sheath that provides an expandable accessory lumen.

FIG. 14 is a cross-sectional side view of a distal portion of a catheter, similar to the catheter of FIG. 2, having a steering adjustment guide therein.

FIG. 15 is a cross-section of the catheter of FIG. 14, taken along line 14-14.

FIGS. 16A and 16B are side views of the distal portion of the catheter of FIG. 14, with the steering adjustment guide directed to change a radius of curvature of the distal portion.

FIG. 17 is a perspective view of another embodiment of a catheter (with a portion of the catheter removed for clarity), including a steering element and a steering adjustment member.

FIG. 18 is a cross-sectional view of the catheter of FIG. 17 taken along line 18-18.

FIG. 19 is a cross-sectional view of an alternative embodiment of a catheter including steering element and steering adjustment member within a keyhole lumen within the catheter.

FIG. 20 is an exploded cross-sectional view of another alternative embodiment of a catheter including steering element and steering adjustment member within a keyhole lumen within the catheter.

FIG. 21A is an exploded cross-sectional view of yet another alternative embodiment of a catheter including steering element and steering adjustment member within a keyhole lumen within the catheter.

FIG. 21B is a detail of the keyhole lumen of FIG. 21A with the steering element and steering adjustment member received therein.

FIG. 22 is a perspective view of still another embodiment of a catheter (with a portion of the catheter removed for clarity), including a steering element and a steering adjustment member.

FIG. 23 is a cross-sectional view of the catheter of FIG. 22 taken along line 23-23.

FIGS. 24A-24F are cross-sections of alternative embodiments of a steering adjustment member that may be provided in a catheter.

FIGS. 25A and 25B are cross-sectional views of another embodiment of a catheter including a steering adjustment member that may create a hinge biasing the catheter to bend in a desired direction.

FIGS. 26 and 27 are cross-sectional views of additional embodiments of catheters including steering elements and steering adjustment members.

FIG. 28 is a cross-sectional side view of another embodiment of a catheter including a steering element and steering adjustment member therein.

FIG. 29 is a perspective detail of a distal tip of the steering adjustment member of FIG. 28 receiving the steering element therethrough.

FIGS. 30A and 30B are perspective views of another embodiment of a catheter (partially omitted for clarity) including multiple steerable regions in straight and bent configurations, respectively.

FIGS. 31A and 31B are cross-sections of the catheter of FIGS. 30A and 30B taken along lines 31A-31A and 31B-31B, respectively.

FIG. 32A and 32B are side views of yet another embodiment of a catheter including multiple steerable regions in straight and bent configurations, respectively.

FIGS. 33A-33C are cross-sections of the catheter of FIGS. 32A and 32B, taken along lines 33A-33A, 33B-33B, and 33C-33C, respectively.

FIG. 34 is a perspective view of a proximal end of a steerable guidewire, including a steering element and steering adjustment member.

FIG. 35 is a perspective view of a clamshell actuation member that may be received around the proximal end of the guidewire of FIG. 34.

FIG. 36 is a cross-sectional side view of the clamshell of FIG. 35 received around the guidewire of FIG. 34.

FIGS. 37-39 are perspective views of an apparatus for receiving and/or advancing a guidewire into an intravascular device.

FIGS. 40-44 are alternate embodiments of the apparatus of FIGS. 37-39.

FIG. 45 is a perspective view of the apparatus of FIG. 44 receiving a steerable catheter therethrough.

FIG. 46A is a side view of a distal portion of another embodiment of a steerable catheter.

FIG. 46B is an exploded side view of the steerable catheter of FIG. 46A.

FIG. 47 is a cross-section of the steerable catheter of FIG. 46B, taken along line 47-47.

FIGS. 48A and 48B are side views of a distal portion of a steerable catheter including an external steering element, deactivated and activated, respectively.

FIGS. 49A and 49B are cross-sections of the steerable catheter of FIGS. 48A and 48B, taken along lines 49A-49A and 49B-49B, respectively.

FIGS. 50A and 50B are side views of a distal portion of another embodiment of a steerable catheter including an external steering element, deactivated and activated, respectively.

FIGS. 51A and 51B are cross-sections of the steerable catheter of FIGS. 50A and 50B, taken along lines 51A-51A and 51B-51B, respectively.

FIG. 52 is a side view of a catheter carrying a balloon and imaging assembly for imaging through the balloon.

FIG. 53A is a side view of a catheter carrying a balloon that has "puckered" to create a pocket for trapping blood.

FIG. 53B is a side view of a catheter carrying a balloon in which the balloon is aligned with and abuts a coronary sinus.

FIG. 53C is a side view of a catheter including an embodiment of a balloon having a geometry that displaces blood from the field of view while minimizing balloon volume.

FIG. 53D illustrates a correlation between field of view (FOV) and balloon size.

FIG. 54 is a side view of a catheter including a balloon, illustrating blood trapped between the balloon and tissue against which the balloon is directed.

FIG. 55 is an image of a field of view of the balloon of FIG. 54 illustrating a central opaque region created by trapped blood.

FIG. 56 is a side view of a catheter including a balloon having a lumen therein provide an egress path for blood.

FIG. 57 is a side view of another embodiment of a catheter including a balloon and having a lumen communicating with an exit port to provide an egress path.

FIG. 58 is a side view of yet another embodiment of a catheter including balloon including a plurality of grooves providing an egress path.

FIG. 59 is a perspective view of a distal tip of another embodiment of an apparatus including a balloon and imaging assembly.

FIG. 60 is an end view of the distal tip of FIG. 59, showing exemplary ray lines of the imaging assembly.

FIG. 61 illustrates a view from a camera or other imaging device showing a view of an illuminated area imaged with the imaging assembly of FIG. 60.

## DETAILED DESCRIPTION OF THE PREFERRED EMBODIMENTS

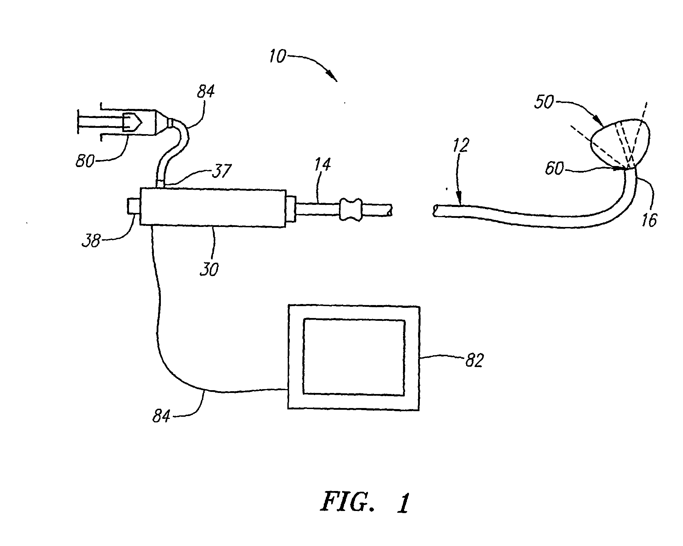

Turning to the drawings, FIG. 1 shows a first embodiment of an apparatus 10 for imaging a body lumen, e.g., for visualizing, accessing, and/or cannulating a body lumen from a body cavity (not shown). As explained further below, the apparatus 10 may be used for imaging a wall of a body lumen, e.g., a right atrium of a heart, e.g., for visualizing, accessing, and/or cannulating a coronary sinus ostium. Alternatively, the apparatus 10 may be used for visualizing, accessing, and/or cannulating other body lumens, e.g., for delivering one or more therapeutic and/or diagnostic agents into tissue, and/or for puncturing through tissue to access a region beyond the punctured tissue. As used herein, "body lumen" may refer to any passage within a patient's body, e.g., an artery, vein, or other blood vessel, or a body cavity, such as a chamber within a patient's heart, e.g., a ventricle or atrium. Although exemplary embodiments are described herein, additional information that may relate to the structure and/or methods for making and/or using the apparatus 10 may also be found in co-pending application Serial No. 10/447,526, filed May 29, 2003.

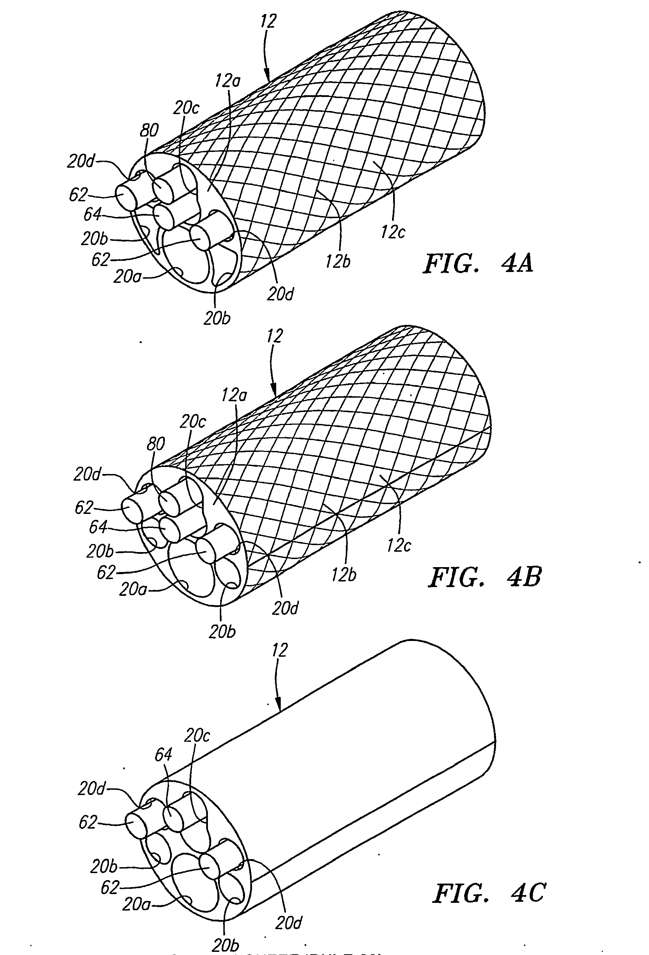

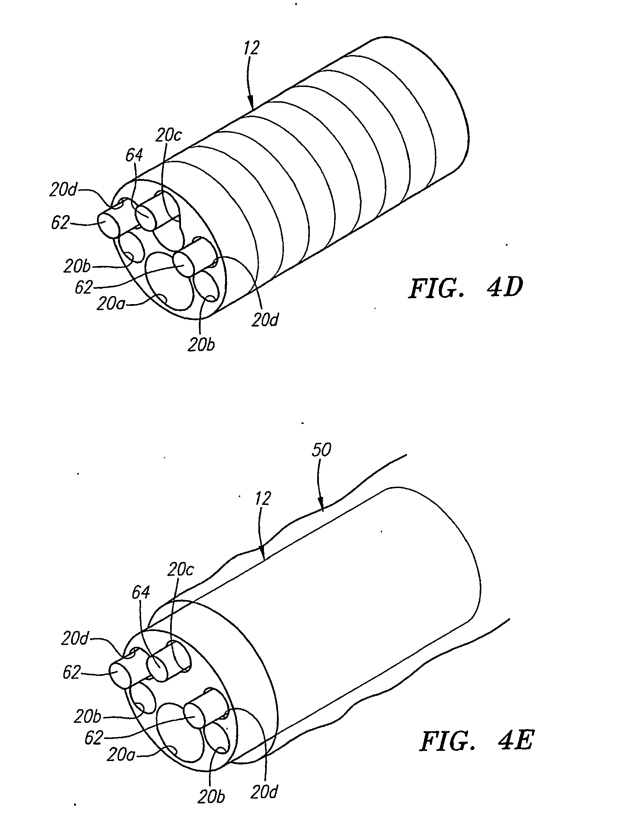

Generally, as shown in FIG. 1, the apparatus 10 includes a catheter or other elongate member 12, including a handle 30 on a proximal end 14 of the catheter 12, and a balloon or other expandable member 50 on a distal end 16 of the catheter 12. An imaging assembly 60 may be provided on or otherwise carried by the catheter 12 for imaging through the balloon 50, e.g. including one or more illumination fibers 62 and/or imaging optical fibers 64 (not shown in FIG. 1, see, e.g., FIGS. 4A-4E) extending through the catheter 12, as described further below. Optionally, the apparatus 10 may include other components, e.g., a syringe or other source of inflation media 80, a monitor or other output device 82, and the like. In additional embodiments, the apparatus 10 may include other devices that may be delivered through, over (e.g., a sheath over the catheter 12), or otherwise advanced from the catheter 12, e.g., a guidewire, a needle, a guide catheter, an energy probe, and the like (not shown), as described further below.

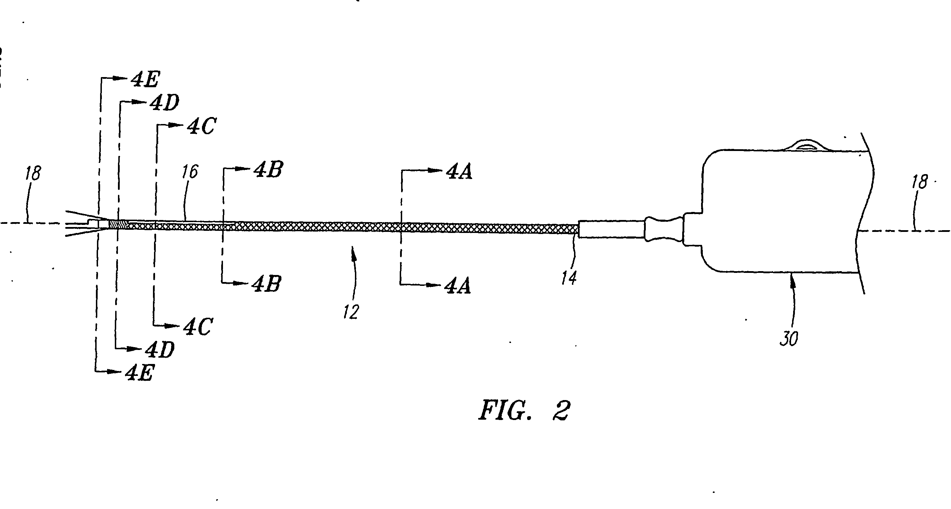

Turning to FIG. 2, the catheter 12 generally is an elongate tubular body including a proximal end 14, a distal end 16 having a size and shape for insertion into a patient's body, and a central longitudinal axis 18 extending between the proximal and distal ends 14, 16. As shown in FIGS. 4A-4E, the catheter 12 may include one or more lumens 20 extending between the proximal and distal ends 14, 16, e.g., an accessory lumen 20a, one or more inflation lumens 20b (two shown), and one or more lumens 20c, 20d for the imaging assembly 60. Optionally, the catheter 12 may include one or more additional lumens (not shown) extending at least partially between the proximal and distal ends 14, 16, e.g., for one or more separate steering elements (not shown). In exemplary embodiments, the catheter 412 may have a diameter between about four and ten French (1.33-3.33 mm), or between about six and eight French (2.00-2.67 mm). In alternative embodiments, the catheter 12 may be used as a guidewire, e.g., having a diameter of not more than about 0.014 inch (0.35 mm) or less.

The catheter 12 may be substantially flexible, semi-rigid, and/or rigid along its length, and may be formed from a variety of materials, including plastic, metal, and/or composite materials. For example, the catheter 12 may be substantially flexible at the distal end 16, e.g., to facilitate steering and/or advancement through tortuous anatomy, and/or may be semi-rigid or rigid at the proximal end 14, e.g., to enhance pushability of the catheter 12 without substantial risk of buckling or kinking. In an exemplary embodiment, the catheter 12 may be formed from PEBAX, which may include a braid or other reinforcement structure therein. For example, as shown in FIGS. 4A and 4B, the catheter 12 may include a plastic core 12a, e.g., polyurethane, extruded or otherwise formed with the lumens 20 therein, over which a braid 12b, e.g., of metal, plastic, or composite fibers, may be disposed. A tube of PET 12c (partially cut away in FIG. 4B) may be disposed around the braid-covered core 12a, and then heat shrunk or otherwise attached to capture and/or secure the braid 12b between the tube 12c and the core 12a. Optionally, an adhesive may be used to bond one or more of the layers 12a-12c of the catheter 12 together.

Furthermore, the plastic core may be composite construction. For example, a portion of catheter 12 adjacent the distal end 16 may include semi-cylindrical portions of different materials that may be secured together to provide a tubular body. For example, the last several inches, e.g., up to five inches, adjacent to the distal end 16 may include an upper and lower halves or portions (not shown) that may be bonded or otherwise secured together. The upper half (containing the imaging fibers 62, 64) may be an extrusion made from polyurethane, and the lower half (containing the accessory lumen 20a and/or inflation lumens 20b) may be made from PEBAX. This may provide a desired center of modulus or hinge, as explained further below.

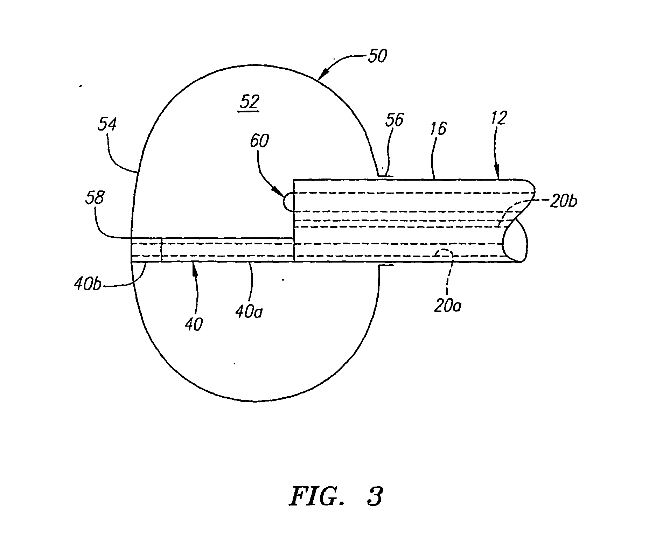

Optionally, with additional reference to FIG. 3, the catheter 12 may include a tubular extension 40 that extends distally from the distal end 16. The tubular extension 40 has a diameter or other cross-section that is substantially smaller than the catheter 12. In addition, the tubular extension 40 may be offset from or concentric with the central axis 18 of the catheter 12. The tubular extension 40 may facilitate balloon stabilization and/or may maximize a field of view of the imaging assembly 60, as explained further below. The tubular extension 40 may include a section of hypotube or other tubular material, e.g., formed from metal, plastic, or composite materials. In an exemplary embodiment, the tubular extension 40 may include a first section 40a formed from a substantially rigid material, e.g., stainless steel, and a second tip section 40b formed from a flexible material, e.g., PEBAX, to provide a relatively soft and/or atraumatic tip for the apparatus 10. Such a tip section 40b may reduce abrasion or other tissue damage while moving the tubular extension 40 along tissue during use, as explained further below.

The first section 40a may be at least partially inserted into the distal end 16 of the catheter 12, e.g., into the accessory lumen 20a. For example, the material of the distal end 16 may be softened to allow the material to reflow as the first section 40a of the tubular extension is inserted into the accessory lumen 20a. Alternatively, the distal end 16 may include a recess (not shown) sized for receiving a portion of the first section 40a therein. In addition or alternatively, the first section 40a may be attached to the distal end 16 by bonding with adhesive, using mating connectors and/or an interference fit, and the like. The second section 40b may be bonded or otherwise attached to the first section 40a before or after the first section 40a is attached to the distal end 16 of the catheter 12.

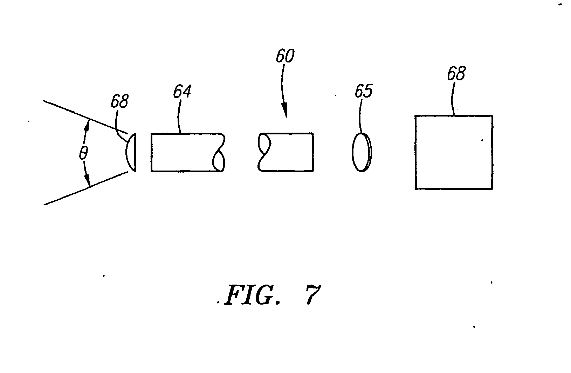

Turning to FIGS. 1 and 7, with additional reference to FIGS. 4A-4E, the imaging assembly 60 generally includes an objective lens 66, e.g., a gradient index ("GRIN") lens, self-oc lens, or other optical imaging element, that is exposed within an interior 52 of the balloon 50 for capturing light images through the balloon 50. The objective lens 66 may be coupled to an optical imaging fiber 64, e.g. a coherent image bundle, that extends between the proximal and distal ends 14, 16 of the catheter 12, e.g., through the lumen 20d, as shown in FIGS. 4A-4E.

In one embodiment, the objective lens 66 may have a diameter similar to the imaging fiber 64, e.g., to simplify bonding and/or alignment, and/or to decrease its overall profile. For example, the objective lens 66 may have a diameter of not more than about three hundred fifty and five hundred microns (350-500 $\mu$m). Exemplary lenses may be available from Nippon Sheet Glass ("NSG") or Grintech.

The objective lens 66 may focus reflected light from images obtained through the balloon 50 onto the face of the imaging fiber 64. The objective lens 66 may have a relatively large numerical aperture (NA), determined by:

$$NA = \sin(\Theta/2).$$

Where Θ is the view angle of the lens 66, as shown in FIG. 7. Alternatively, a wide angle lens may be provided for the objective lens 66 to increase the functional numerical aperture. Optionally, the objective lens 66 may be coated, e.g., to reduce surface reflection and/or otherwise optimize optical properties.

The imaging fiber 64 may include a plurality of individual optical fibers, e.g., between about one thousand and one hundred fifty thousand (1,000-150,000) fibers, or between about three thousand and ten thousand (3,000-10,000) fibers, in order to provide a desired resolution in the images obtained by the optical fiber 64. The material of the imaging fiber 64 may be sufficiently flexible to bend as the catheter 12 bends. Optionally, the imaging fiber 64 may be leached to increase its flexibility.

A device 68 may be coupled or otherwise provided at the proximal end 14 of the apparatus 10 for acquiring, capturing, and/or displaying images transmitted by the imaging fiber 64. As shown in FIG. 7, one or more lenses 65 may be coupled to the fiber bundle 64 for focusing and/or resolving light passing through the imaging fiber 64, e.g., to pass the image to the device 68. The lens 65 may be coupled directly between the imaging fiber 64 and the device 68 or may be spaced apart from one or both the imaging fiber 64 and the device 68. The lens 65 should provide sufficient magnification to prevent substantial loss of resolution, which may depend upon the pixel density of the device 68. For example, a lens 65 having magnification between about 1.3x and 3x may spread a single pixel from the optical fiber 64 onto four or more pixels on the device 68, which may sufficiently reduce resolution loss.

The device 68 may include a CCD, CMOS, and/or other device, known to those skilled in the art, e.g., to digitize or otherwise convert the light images from the imaging fiber 64 into electrical signals that may be transferred to a processor and/or display. The device 68 may be a color device, or may be black and white, which may increase sensitivity. The smaller the pixel size of the device 68, the less magnification that may be needed by the lens 65. In exemplary embodiments, the device 68 may have pixel sizes between about one and ten microns (1-10 μm), or between about two and five microns (2-5 μm).

The device 68 may be coupled to a monitor 82, e.g., by a cable 84, as shown in FIG. 1. In addition or alternatively, a computer or other display or capture devices (not shown) may be coupled to the device 68 to display and/or store the images acquired from the imaging fiber 64. Additional information on capture devices that may be used may be found in application Serial No. 10/447,526.

The imaging assembly 60 may also include one or more illumination fibers or light guides 62 carried by the distal end 16 of the catheter 12 for delivering light into the interior 52 and/or through a distal surface 54 of the balloon 50. As shown in FIGS. 4A-4E, a pair of illumination fibers 62 may be provided in the catheter 12.

The illumination fibers 62 may be spaced apart from one another, e.g., in separate lumens 20d to minimize shadows, which may be cast by the tubular extension 40. A source of light (not shown) may be coupled to the illumination fiber(s) 62, e.g., via or within the handle 30, for delivering light through the illumination fiber(s) 62 and into the balloon 50.

Optionally, the catheter 12 may be steerable, i.e., the distal end 16 may be controllably deflected transversely relative to the longitudinal axis 18 using one or more pullwires or other steering elements. In the embodiment shown in FIGS. 4A-4E, the imaging fiber 64 may be used for steering the distal end 16 of the catheter 12 in one transverse plane (thereby providing one degree of freedom), as well as for obtaining images through the balloon 50. Alternatively, multiple pullwires (not shown) may be provided for steering the distal end 16 of the catheter 12 in two or more orthogonal planes (thereby providing two or more degrees of freedom).

The imaging fiber 64 (or other pullwire, not shown) may be attached or otherwise fixed relative to the catheter 12 at a location adjacent the distal end 16, offset radially outwardly from a center of modulus of the catheter 12. If the construction of the catheter 12 is substantially uniform about the central axis 18, the center of modulus may correspond substantially to the central axis 18. If the construction of the catheter 12 is asymmetrical about the central axis 18, however, the center of modulus may be offset from the central axis 18 in a predetermined manner. As long as the optical fiber 64 (or other pullwire) is fixed at the distal end offset radially from the center of modulus, a bending moment will result when the imaging fiber 64 is pushed or pulled relative to the catheter 12 to steer the distal end 16.

For example, when the optical fiber 64 is pulled proximally or pushed distally relative to the catheter 12, e.g., from the proximal end 14 of the catheter 12, a bending force may be applied to the distal end 16, causing the distal end 16 to curve or bend transversely relative to the central axis 18. Optionally, as described further below, the degree of steerability of the distal end 16 may be adjustable, e.g., to increase or decrease a radius of curvature of the distal end 16 when the imaging fiber 64 is subjected to a predetermined proximal or distal force. In addition or alternatively, one or more regions of the catheter 12 may be set to be steerable in a predetermined manner.

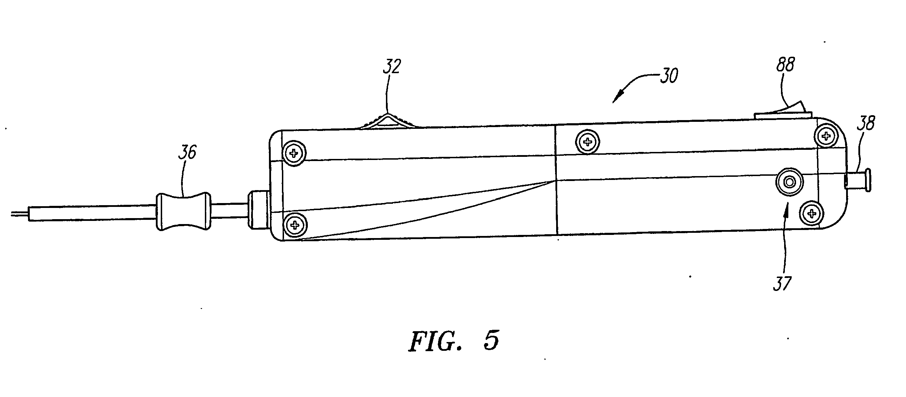

Turning to FIG. 5, the handle 30 may be an enlarged member coupled to or otherwise provided on the proximal end 14 of the catheter 12. The handle 30 may be contoured or otherwise shaped to facilitate holding the handle 30 and/or otherwise manipulating the catheter 12. The handle 30 may be formed from one or more parts of plastic, metal, or composite material, e.g., by injection molding, and the like, that may be assembled together, e.g., using mating connectors, adhesives, and the like.

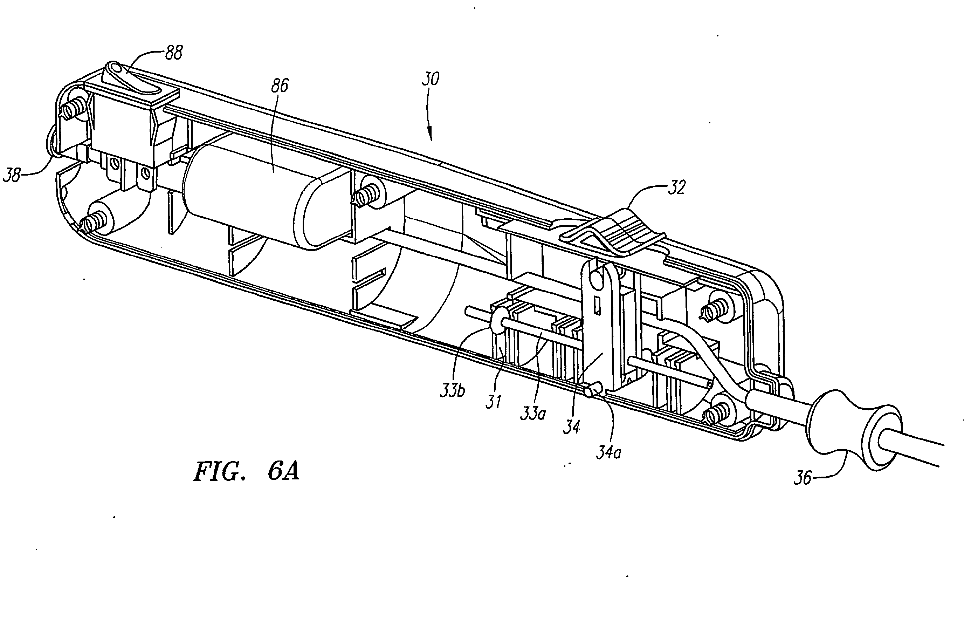

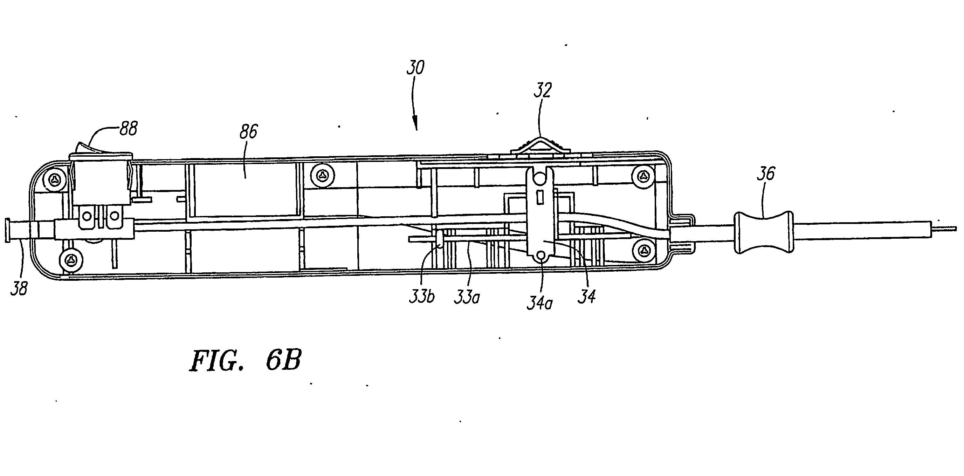

The handle 30 may include one or more steering controls 32, 34 for controlling the ability to steer the distal end 16 of the catheter 12. For example, as shown in FIGS. 6A and 6B, the handle 30 may include an actuator 32 that may be coupled to the optical fiber 64 (not shown in FIGS. 6A-6B) via a linkage 34. The linkage 34 may be pivotally coupled to the handle 30 by a pin 34a such that proximal movement of the actuator 32 causes the linkage 34 to apply a proximal force to the optical fiber 64. The resulting bending moment causes the distal end 16 of the catheter 12 to bend into a curved shape, such as that shown in FIG. 1. This mechanism may also include mechanical advantage.

Optionally, the actuator 32 may be biased, e.g., to return the distal end 16 of the catheter 12 to a generally straight configuration when the actuator 32 is released. For example, as shown in FIGS. 6A and 6B, the linkage 34 may be coupled to a resistive mechanism 33 that may allow the actuator 32 to be moved by applying a proximal force to overcome the resistance of the resistive mechanism 33. When a proximal force is removed, e.g., when the actuator 32 is released, the resistive mechanism 33 may return the linkage 34, and consequently the actuator 32 and imaging fiber 64 to a neutral position, thereby substantially straightening the distal end 16 of the catheter 12.

In another embodiment, the resistive mechanism 33 may allow the distal end 16 to maintain a curved configuration once the actuator 32 is moved to steer the distal end 16. As shown in FIGS. 6A and 6B, the resistive mechanism 33 includes a section of tubing 33a coupled to a flexible o-ring 33b that is substantially fixed relative to the handle 30. The o-ring 33b may be secured within a pocket 31 in the handle 30 to prevent the o-ring 33b from moving substantially. The o-ring 33b may be sufficiently flexible to allow the tubing 33a to slide axially through the o-ring 33b when the actuator 32 is pulled, yet may apply a predetermined resistance to such axial movement. Thus, when the actuator 32 is actuated, the resistance of the o-ring 33b may be overcome to cause the distal end 16 of the catheter 12 to curve. When the actuator 32 is released, the o-ring 33b may apply a desired friction against the tubing 33a, thereby preventing the tubing 33a from moving, and consequently maintaining the set curve of the distal end 16. To curve the distal end 16 further or partially or entirely straighten the distal end 16, the actuator 32 may be slid further proximally or distally to overcome the resistance provided by the o-ring 33b. Additional steering elements and structures and methods for using them are disclosed in application Serial No. 10/447,526.

In addition, the handle 30 may include a slider 36 for controlling a variable steering radius ("VSR") mechanism carried by the distal end 16 of the catheter 12. The VSR mechanism may change the radius of curvature of the distal end 16 when the actuator 32 is activated and/or the region of the distal end 16 that is steered, depending upon the relative position of the slider 36. For example, as explained further below, when the slider 36 is in a proximal position, e.g., immediately adjacent the handle 30, the bending moment created when the actuator 32 is activated may be maximized, thereby resulting in a relatively large radius of curvature when the distal end 16 is steered. As the slider 36 is directed distally, the radius of curvature of the distal end 16 may become smaller and more distal.

The handle 30 may also include ports, seals, and/or other connections for connecting other components to the catheter 12 and/or introducing one or more accessories into the catheter 12. For example, as shown in FIG. 5, a port 37 may be provided that communicates with the inflation lumen(s) 20b of the catheter 12 (not shown, see FIGS. 4A-4E). A luer lock or other connector may be provided on the port 37 for temporarily connecting tubing or other fluid-conveying components to the handle 30. As shown in FIG. 1, a syringe or other source of fluid 80, e.g., including saline, carbon dioxide, nitrogen, or air, may be connected to the port 37 via tubing 84 to the inflation lumens 20b of the catheter 12, e.g., for expanding the balloon 50 when fluid is delivered into an interior 52 of the balloon 50. Alternatively, the syringe 80 may be a source of vacuum, e.g., for collapsing the balloon 50 when fluid is evacuated from the interior 52.

Similarly, an access port 38 may be provided that communicates with the accessory lumen 20a of the catheter 12 (also not shown, see FIGS. 4A-4E). Optionally, the access port 38 may include a connector, e.g., a luer lock, and/or one or more seals, e.g., a hemostatic seal, allowing one or more instruments (such as a guidewire, a needle, a guide catheter, and/or an energy probe, not shown) to be inserted through the access port 38 and into the accessory lumen 20a. Alternatively, another source of fluid, e.g., saline, and/or one or more therapeutic or diagnostic agents (not shown), may be connectable via tubing (also not shown) to the accessory lumen 20a, e.g., for delivering fluid beyond the distal end 16 of the catheter 12.

Optionally, the handle 30 may include other components, e.g., a battery or other power source 86, a light source (not shown), e.g., one or more light emitting diodes ("LEDs") that may be coupled to the illumination fiber(s) 62 for transmitting light beyond the distal end 16 of the catheter 12. In addition, the handle 30 may include a switch 88, e.g., for turning electrical components of the handle 30 on and off, such as the light source.

Returning to FIGS. 1 and 3, a substantially transparent balloon 50 may be provided on the distal end 16 of the catheter 12. The balloon 50 may be expandable from a contracted condition (not shown, see, e.g., FIG 4E) to an enlarged condition (as shown in FIG. 3), e.g., when fluid is introduced into the interior 52 of the balloon 50. The balloon 50 may be formed from one or more compliant and/or elastomeric materials, such as silicone, latex, or other synthetic or natural elastomers, such as those sold under the trade names Isoprene or Chronoprene. Alternatively, the balloon 50 may be formed from substantially noncompliant material, e.g., polytetrafluoroethylene (PTFE), expanded polytetrafluoroethylene (EPTFE), fluorinated ethylenepropylene (FEP), polyethylene teraphthalate (PET), urethane, olefins, and polyethylene (PE), such that the balloon 50 may expand to a predetermined shape when fully inflated to the enlarged configuration.

In the enlarged condition, the balloon 50 may have a distal surface 54 that is substantially flat or otherwise configured for contacting a wall of a body cavity, such as the right atrium (not shown). The balloon 50 may have a generally spherical shape, a frusto-conical shape, and the like, thereby defining the distal surface 54 beyond the distal end 16 of the catheter 12.

The balloon material may be sufficiently flexible and/or elastic such that the distal surface 54 may conform substantially to the wall of a body cavity. The balloon 50 may also be sufficiently noncompliant to displace blood or other fluid from between the distal surface 54 and the wall of the body cavity to facilitate imaging tissue of the wall through the balloon 50, as explained further below. The balloon 50 may be molded around or within a mold (not shown) having a desired shape for the balloon 50 in the enlarged or contracted condition. Alternatively, the balloon 50 may be formed from one or more panels that may be attached to one another, e.g., using an adhesive (such as an adhesive cured using ultraviolet ("UV") light), sonic welding, and/or heating, after lapping or butting adjacent panels together.

The balloon 50 may include a proximal end 56 that may be attached to an outer surface of the catheter 12 adjacent the distal end 16, e.g., using an adhesive, heating, sonic welding, an interference fit, and/or an outer sleeve or other wrap (not shown). The distal surface 54 of the balloon 50 may include an opening 58 therein, allowing the balloon 50 to be bonded or otherwise attached to the tubular extension 40 around the opening 58. In one embodiment, the distal surface 54 of the balloon 50 may extend slightly beyond the tip 40b of the tubular extension 40 to enhance the atraumatic character of the apparatus 10 when the balloon 50 is directed against tissue.

As shown in FIG. 3, the interior 52 of the balloon 50 may communicate with the inflation lumen(s) 20b of the catheter 12. Substantially transparent inflation media, e.g., saline, carbon dioxide, nitrogen, air, and the like, may be introduced into the interior 52 of the balloon 50, e.g., from the syringe 80 shown in FIG. 1, to expand the balloon 50 towards the enlarged condition. As used herein, "transparent" refers to any material and/or fluid that may permit sufficient light to pass therethrough in order to identify or otherwise visualize objects through the material and/or fluid. "Light" as used herein may refer to light radiation within the visible spectrum, but may also include other spectra, such as infrared ("IR") or ultraviolet ("UV") light.

Alternatively, the balloon 50 may be provided using different configurations, materials, and/or methods, such as those disclosed in co-pending application Serial No. 10/447,526.

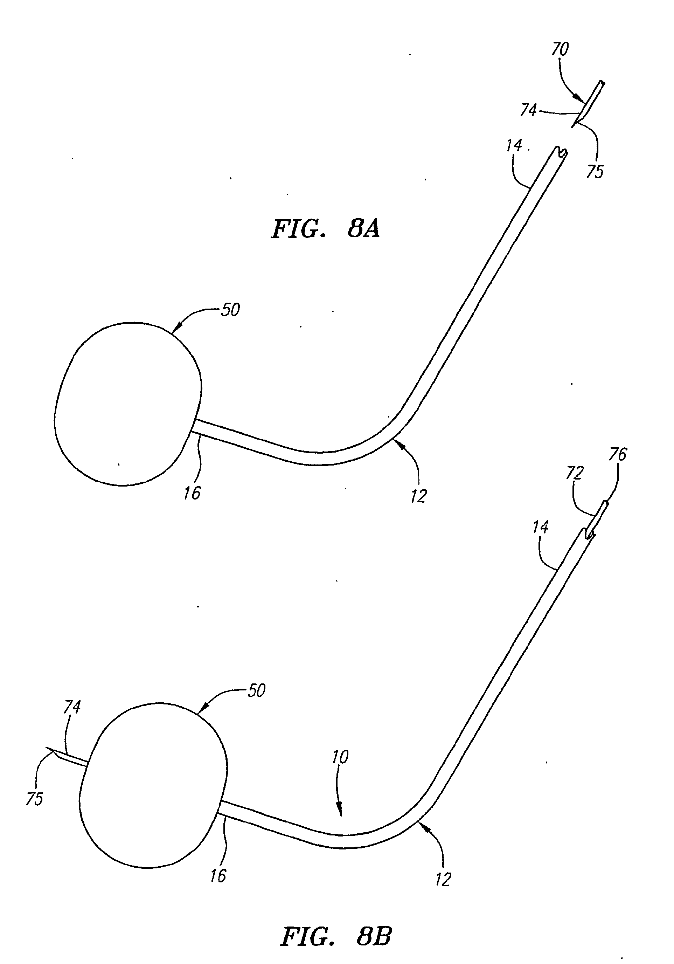

Turning to FIGS. 8A and 8B, the apparatus 10 may include a needle 70 that may be deployable from the distal end 16 of the catheter 12. The needle 70 generally includes a proximal end 72, a distal end 74 sized for insertion into the accessory lumen 20a of the catheter 12 and terminating in a sharpened distal tip 75, and a lumen 76 extending between the proximal and distal ends 72, 74. As shown, the needle 70 is advanceable substantially axially through the accessory lumen 20a of the catheter 12, and consequently, through the tubular extension 40 and beyond the distal surface 54 of the balloon 50. The needle 70 may be formed from stainless steel or other material having sufficient flexibility to be advanced through the catheter 12, e.g., when the catheter 12 has been advanced through tortuous anatomy, yet have sufficient rigidity to be advanced through tissue. The needle 70 may have a single distal opening, or an array of openings (not shown) may be provided in the distal tip 75 for delivering fluid in a desired manner from the distal tip 75. Exemplary configurations of needles and that may be used in association with the apparatus 10 and methods for treating tissue with such needles are disclosed in U.S. Patent No.6,283,951.

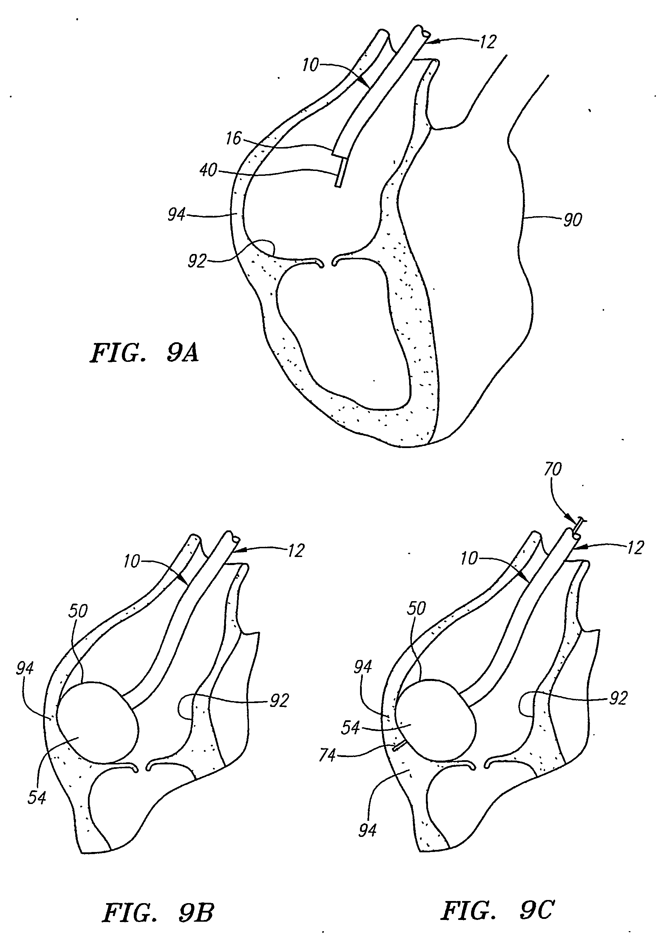

Turning to FIGS. 9A-9C, a method not forming part of the invention, is shown for delivering one or more therapeutic and/or diagnostic agents into tissue within a patient's heart. For example, the apparatus 10 may be used for delivering stem cells into tissue, e.g., that has undergone necrosis after an acute myocardial infarction. It has been found that injecting necrotic tissue with stem cells may restore contractility to large volumes of heart tissue. However, because of the scarcity and cost of stem cells, the stem cells should only be delivered into necrotic tissue and not into otherwise healthy tissue where it is not needed.

The distal end 16 of the apparatus 10 may be introduced into a patient's body using conventional methods used for delivering catheters or other instruments. For example, with the balloon 50 collapsed, the distal end 16 of the catheter 12 may be introduced into a patient's vasculature, e.g., from a percutaneous puncture, e.g., in a peripheral vessel, such as a femoral artery or vein, carotid artery, and the like, depending upon which side of the heart is to be treated. For example, as shown in FIG. 9A, if tissue within the right atrium 92 of the heart 90 is to be treated, the catheter 12 may be introduced through the venous system into the superior or inferior vena cava (superior approach being shown in FIG. 9A) and into the right atrium 92.

Turning to FIG. 9B, once within the right atrium 92, the balloon 50 may be expanded, and the apparatus 10 may be manipulated to place the distal surface 54 of the balloon 50 into contact with the wall 94 of the heart 90 within the right atrium 92. Optionally, this manipulation may involve steering the distal end 16 of the apparatus 50, e.g., using one or more pullwires or other steering mechanisms actuated from the proximal end (not shown) of the apparatus 10, as described elsewhere herein.

In addition or alternatively, other imaging systems may be used to monitor the apparatus 10 to facilitate introducing the apparatus 10 into the heart 90. For example, external imaging systems, such as fluoroscopy, ultrasound, magnetic resonance imaging (MRI), and the like, may provide feedback as to the location and/or relative position of the distal end 16 of the apparatus 12. The distal end 16 may include one or more markers, e.g., radiopaque bands and the like (not shown), that may facilitate such imaging. External imaging may ensure that the apparatus 10 is generally oriented towards a target tissue structure before optical images are acquired and/or the apparatus 10 is manipulated more precisely.

With the distal surface 54 of balloon 50 placed against the wall 94 of the heart 90, the imaging assembly 60 (not shown, see, e.g., FIG. 7) of the catheter 12 may be activated to image the wall 94. Sufficient distal force may be applied to the apparatus 10 to squeeze blood or other fluid from between the distal surface 54 and the wall 94, thereby clearing the field and facilitating imaging the wall 94. Optionally, a substantially transparent fluid, e.g., saline, may be delivered through the catheter 12 (e.g., through accessory lumen 20a, not shown) and the tubular extension 40 to further direct blood or other fluid away from the distal surface 54 of the balloon 50 or otherwise clear the field of view of the imaging assembly 60.

Using the imaging assembly 60 to directly visualize the wall 94, the apparatus 10 may be moved along the wall 94 until a target structure is within the field of view. For example, tissue that has undergone necrosis changes color compared to otherwise healthy tissue, while scar tissue may appear white and/or shiny compared with healthy tissue. In addition, areas around damaged tissue may become hyperemic with increased blood flow. Using the imaging assembly 60 on the catheter 12 to distinguish necrotic tissue from healthy tissue, e.g., using the indicators just identified, necrotic tissue along the wall 94 may be identified for treatment.

Once a target tissue region has been identified for treatment using the imaging assembly 60, the apparatus 10 may be moved further, e.g., until the target tissue region is centered in the field of view or otherwise oriented in a desired manner relative to the tubular extension 40. As shown in FIG. 9C, the distal end 74 of the needle 70 may then be advanced from the distal end 16 of the catheter 12 to puncture and enter at least partially into the target tissue region. The needle 70 may be carried within the catheter 12 while the catheter 12 is introduced with the distal end 74 retracted within the distal end 16 or the needle 70 may be advanced into the catheter 12 after the catheter 12 is introduced into the heart 90 or even after the target tissue region is identified.

If not already provided, a source of stem cells (not shown) may be coupled to the proximal end 72 of the needle 70, and stem cells may be injected through the needle 70 (or through a plurality of needles, not shown, each needle having one or more holes) into the target tissue region. Once sufficient stem cells are delivered, the needle 70 may be retracted back into the distal end 16 of the catheter 12. Optionally, one or more additional regions of necrotic tissue may be identified and stem cells injected therein. Once the desired one or more regions are treated, the balloon 50 may be collapsed, and the apparatus 10 removed from the patient's body.

In other alternatives, one or more additional therapeutic and/or diagnostic agents may be delivered into tissue in addition to or instead of stem cells, similar to the methods just described. In addition, the apparatus 10 may also be used for antegrade or retrograde infusion of one or more agents into other regions of the vasculature under direct visual guidance.

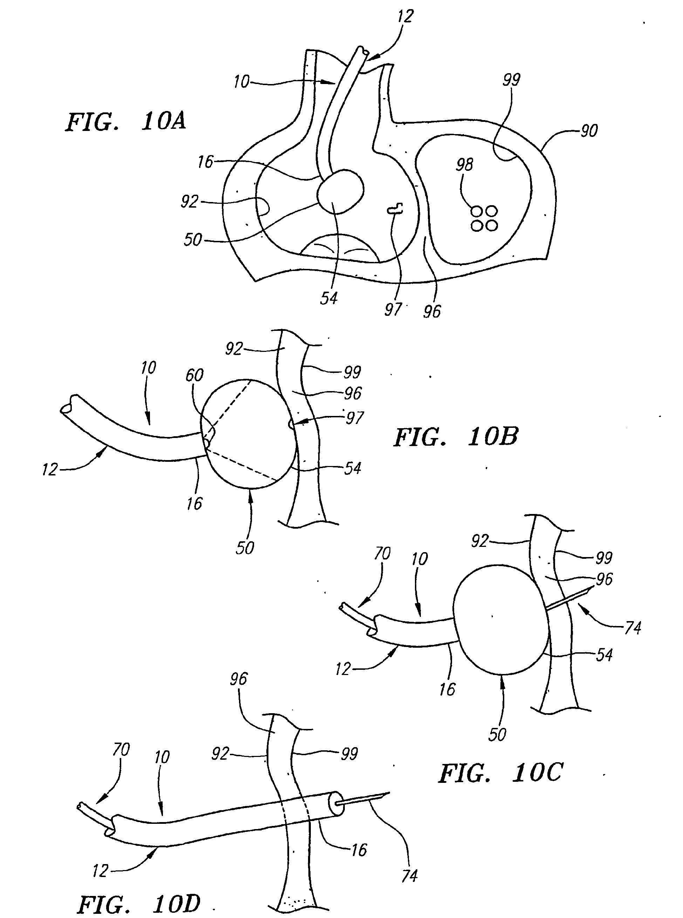

Turning to FIGS. 10A-10C, another method, not forming part of the invention, is shown for treating tissue within a patient's heart. In some procedures, it may be desirable to cross through a septal wall of a heart 90, e.g., the atrial septum 96, since the atria are relatively low pressure regions in the heart. For example, it may desirable to ablate or otherwise deliver electrical energy to tissue surrounding the pulmonary vein ostia 98 located within the left atrium 99, e.g., to treat atrial fibrillation, using access from the right side of the heart 90.

Similar to the previous alternative, initially, the distal end 16 of the catheter 10 may be introduced into the right atrium 92 of the heart 90 with the balloon 50 collapsed (similar to FIG. 9A). Once the distal end 16 is located within the right atrium 92, the balloon 50 may be expanded, as shown in FIG. 10A, and the catheter 12 manipulated to place the distal surface 54 of the balloon 50 into contact with the atrial septum 96 of the heart 90 within the right atrium 92, as shown in FIG. 10B. Optionally, this manipulation may involve steering the distal end 16 of the apparatus 50, similar to the previous methods. Optionally, other imaging systems may be used to monitor the apparatus 10 to facilitate introducing the apparatus 10 into the heart 90 and/or ensure that the apparatus 10 is generally oriented towards the atrial septum 96 before optical images are acquired and/or the apparatus 10 is manipulated more precisely, also similar to the previous embodiments.

With the distal surface 54 of balloon 50 placed against the atrial septum 96 of the heart 90, the imaging assembly 60 may be activated to directly visualize the tissue of the septum 96. Sufficient distal force may be applied to the apparatus 10 to squeeze blood or other fluid from between the distal surface 54 and the septum 96, thereby clearing the field and facilitating imaging the septum 96. Optionally, a substantially transparent fluid, e.g., saline, may be delivered through the catheter 12 (e.g., through accessory lumen 20a, not shown) and the tubular extension 40 to further direct blood or other fluid away from the distal surface 54 of the balloon 50 or otherwise clear the field of view of the imaging assembly 60.

Using the imaging assembly 60 to image the atrial septum 96, the apparatus 10 may be moved along the wall 94 until a target structure is within the field of view. For example, in order to avoid puncturing the heart wall and/or to ensure that the left atrium 99 is accessed, a landmark or other target tissue structure, such as the fossa ovalis ("FOV") 97, may be used to identify an appropriate location to puncture through the septum 96 into the left atrium 99.

Turning to FIG. 10C, once the fossa ovalis 97 (or other target tissue structure) has been identified and/or the apparatus 10 has been properly oriented relative to the fossa ovalis 97, the distal end 74 of the needle 70 may then be advanced from the catheter 12 to puncture the septum 96 and enter into the left atrium 99.

Turning to FIG. 10D, the balloon 50 may then be collapsed, and the distal end 16 of the catheter 12 may be advanced over the needle 70 through the septum 96 and into the left atrium 99. In this embodiment, the distal end 16 of the catheter 12 and/or the tubular extension 40 (if present) may be substantially tapered (not shown) or otherwise configured to facilitate advancing the catheter through the puncture in the septum 96. Once the distal end 16 of the catheter 12 is located within the left atrium 99, the needle 70 may be removed, and an energy probe 100 (or other probe for delivering electrical, light, thermal, or other energy) may be advanced through the accessory lumen 20a of the catheter 12 into the left atrium 99. The probe 100 may be used to ablate or otherwise treat tissue within the left atrium 99. For example, the probe 100 may include one or more electrodes (not shown) that may be used to ablate the ostium of one or more pulmonary veins 98, as is known in the art. A source or ablation energy, e.g., an electrical power generator (not shown), may be coupled to a proximal end of the probe 100, also as is known in the art.

Optionally, the balloon 50 on the catheter 12 may be expanded within the left atrium 99 and the imaging assembly 60 may be used to locate the pulmonary veins 98, using procedures similar to those described above. For example, the balloon 50 may be disposed over the pulmonary vein 98 being treated, whereupon the probe 100 may be advanced through the catheter 12 and the tubular extension 40 into the target ostium. The balloon 50 may remain expanded or may be collapsed when the probe 100 is activated to ablate the ostium of the pulmonary vein 98.

In an alternative, instead of advancing the catheter 12 into the left atrium 99 through the septum 96, a separate guide catheter (not shown) may be advanced over the needle 70 into the left atrium 99. The guide catheter may be advanced through the accessory lumen 20a of the catheter 12 or may be advanced over the entire catheter 12. The probe 100 may then be advanced through the guide catheter (e.g., after removing the needle 70) and manipulated to treat tissue within the left atrium 99.

In an alternative, the apparatus 10 may be used for visualizing the left atrial appendage before delivering an atrial closure device to close the left atrial appendage. For example, the apparatus 10 may be advanced through a puncture in the septum 98 to provide access during a procedure to reduce atrial appendage volume, e.g., using the probe 100. In other alternatives, the apparatus 10 may facilitate removing clots within the left atrium 99, and/or may be used to provide access to permit valve repair and/or replacement. In yet additional alternatives, the apparatus 10 may be used to directly visualize existing defects in a heart, such as atrial or ventricular septal defects. After using the apparatus 10 to identify and locate such defects, a guidewire (not shown) may be advanced through the catheter 12 and into or through the defect, which may facilitate repairing the defect, e.g., by delivering a closure device or otherwise closing the defect.

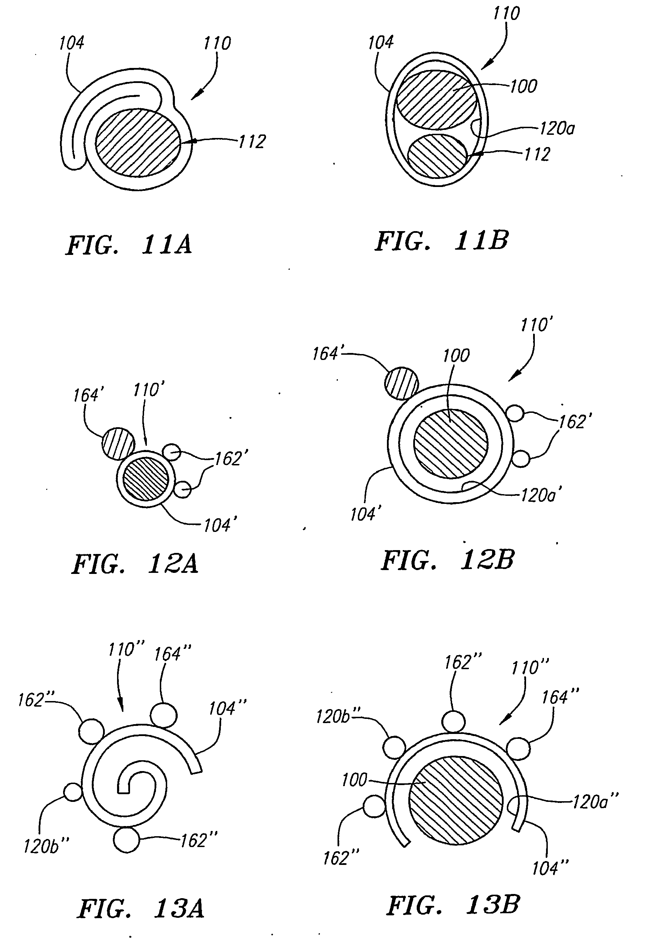

Turning to FIGS. 11A and 11B, in yet another embodiment, an apparatus 110 is shown that may facilitate advancing a guide catheter, energy probe, or other device through a puncture created in a septal wall, as described above. The apparatus 110 may include one or more components similar to the previous embodiments, e.g., a catheter 112 with a handle on a proximal end and a balloon and imaging assembly on a distal end thereof (not shown). Unlike the previous embodiments, the apparatus 110 may include an expandable lumen 120a for receiving an energy probe 100 or other device therethrough. Optionally, to further reduce the profile of the catheter 112, the catheter 112 may not have a pullwire and/or an accessory lumen, as described above with respect to previous embodiments of the catheter 12.

In an exemplary embodiment, the apparatus 110 may include a relatively thin-walled sheath 104 attached to or otherwise extending from an outer surface of the catheter 112. The sheath 104 may be formed from a substantially flexible and/or "floppy" material such that the sheath 104 defines the expandable lumen 120a, yet may be collapsed against or around the catheter 112, as shown in FIG. 11A. When the probe 100 or other device is advanced into the expandable lumen 120a, the sheath 104 partially separate from the catheter 12 and/or otherwise expand to accommodate receiving the device therethrough, as shown in FIG. 11B.

The sheath 104 may be expanded as the probe 100 or other device is inserted into the accessory lumen 120a at the proximal end of the apparatus 110 and is advanced towards the distal end. Alternatively, a fluid or other mechanism may be directed into the accessory lumen 120a to expand the sheath 104 before a device is inserted therein. Thus, the sheath 104 may be similar to the expandable sheaths described in co-pending application Serial Nos. 10/433,321, filed April 24, 2003, 10/934,082, filed September 2, 2004, and 10/958,035, filed October 4, 2004.

The profile of the catheter 112 with the sheath 104 collapsed may be minimized, which may facilitate advancing the catheter 112 through a body lumen, over a needle (not shown), and/or through a puncture, e.g., in a septal wall, similar to the apparatus and methods described above. Once the catheter 112 is disposed through the puncture or septal wall, the probe 100 or other device (not shown), e.g., having a relatively large profile, may be advanced through the accessory lumen 120a of the sheath 104, rather than through a relatively small lumen in the catheter 112. The sheath 104 may facilitate passing the device through the puncture, e.g., dilating the puncture as necessary to accommodate receiving the device therethrough. Once the device is located in the second body cavity, the sheath 104 and/or catheter 112 may be removed from the patient's body, if desired, and the procedure completed similar to the previous embodiments.

Turning to FIGS. 12A and 12B, an alternative embodiment of an apparatus 110' is shown that includes an expandable sheath 104' that may be collapsed to minimize a profile of the apparatus 110' during delivery (as shown in FIG. 12A), and expanded to provide a relatively large accessory lumen 120a' (as shown in FIG. 12B). Unlike the previous embodiments, the sheath 104' may include a braided structure that may collapse to a relatively small cross-section. The braided structure may facilitate expansion and/or otherwise support the sheath 104' during introduction and subsequent use.

The apparatus 110' may include an optical imaging fiber 164' and one or more illumination fibers 162' (two shown), which may be embedded in or otherwise coupled to the sheath 104.' Optionally, the sheath 104' may include other components, e.g., one or more inflation lumens (not shown) that communicate with an interior of a balloon (also not shown) on a distal end of the apparatus 110.' The illumination and imaging fibers 162,' 164' may be substantially fixed when the sheath 104' is in the collapsed condition, thereby allowing tissue to be viewed beyond a distal end of the apparatus 110,' similar to the previous embodiments.

In a further alternative, the sheath 104' may include a membrane, e.g., with or without braids, that may be expanded from the collapsed condition shown in FIG. 12A to an expanded condition shown in FIG. 12B. The lumens or components bonded or otherwise attached to the sheath 104' may be embedded within or attached to an inner or outer surface of the membrane. In one embodiment, the membrane may be an elastomeric material, which may be elastically expandable to accommodate receiving the probe 100 or the device through the accessory lumen 120a.'

Turning to FIGS. 13A and 13B, yet another alternative embodiment of an apparatus 110" is shown that includes a sheath 104" carrying an optical imaging fiber 164," a pair of illumination fibers 162," and an inflation lumen 120b," which may be similar to the previous embodiments. Unlike the previous embodiments, the sheath 104" may be a flat sheet coiled into an overlapping coil extending at least partially between the proximal and distal ends of the apparatus 110." For example, the sheath 104" may be biased to a low profile configuration, e.g., the coiled configuration of FIG. 13A, yet may resiliently unroll to create a relatively large accessory lumen 120a" for receiving an energy probe 100 or other device therein, similar to the previous embodiments.

The apparatus 110" may be introduced into a patient's body in the low profile configuration shown in FIG. 13A. Once within a first body cavity, a balloon (not shown) on the distal end may be expanded, and an imaging assembly (also not shown) may be used to image tissue wall surrounding the first body cavity, e.g., to identify a location to puncture through the wall to a second body cavity, similar to the previous embodiments. Once the location is identified, a needle (not shown) may be advanced through the sheath 104," e.g., through the accessory lumen 120a or through another lumen (not shown) in the wall of the sheath 104." If the accessory lumen 120a is used, the sheath 104" may unroll or otherwise expand partially to accommodate the needle.

The needle may be advanced from the sheath 104" to puncture through the wall of the first body cavity and access the second body cavity. The apparatus 110" may then be advanced over the needle through the puncture into the second body cavity with the balloon collapsed. Within the second body cavity, optionally, the balloon may be expanded again and used to image surrounding tissue to identify a target treatment site, similar to the previous embodiments.

With a target treatment site identified, the probe 100 or other device may be advanced through the accessory lumen 120a," as shown in FIG. 13B, e.g., after withdrawing the needle, and used to treat tissue at the target treatment site, similar to the previous embodiments. Upon completing the procedure, the probe 100 may be removed, whereupon the sheath 104" may resiliently collapse again, facilitating its removal from the patient's body. Alternatively, the probe 100 or other device and apparatus 110" may be removed substantially simultaneously or in other sequences.

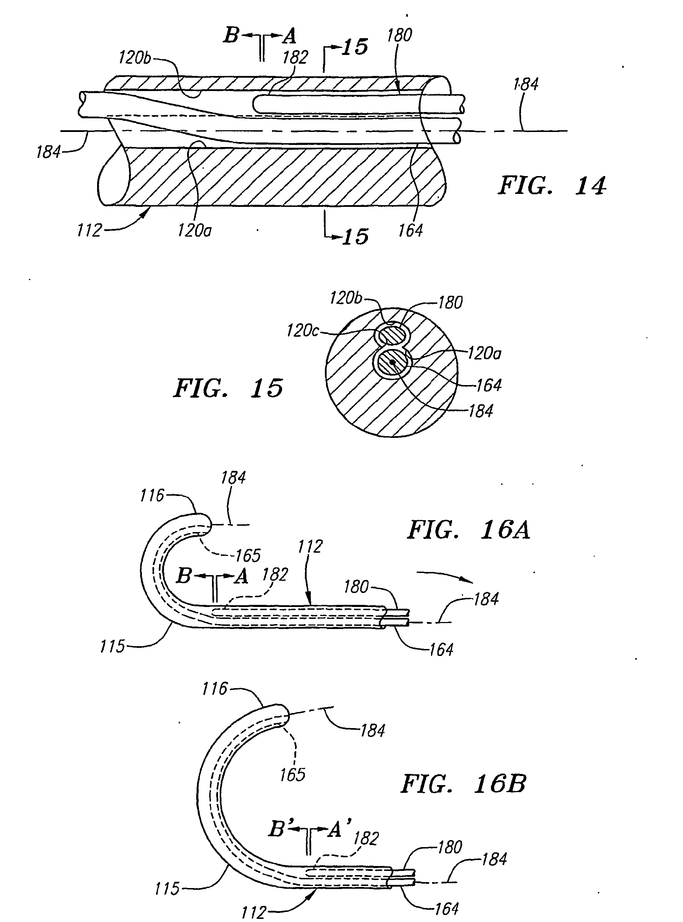

Turning to FIGS. 14 and 15, another embodiment of a catheter 112 is shown that may be similar to the catheter 12 shown in FIG. 2 and described elsewhere herein. Generally, the catheter 112 is an elongate tubular body including a proximal end (not shown), a distal end 116 having a size and shape for insertion into a patient's body, and one or more lumens 120 extending between the proximal end and distal end 116. Although only lumen 120 is shown in FIGS. 14 and 15, it will be appreciated that the catheter 112 may also include one or more additional lumens, e.g., inflation lumens, accessory lumens, and/or lumens for an imaging assembly (not shown for simplicity).

As shown, a steering element 164 and/or a steering adjustment member 180 may be carried by the catheter 112, e.g., within the lumen 120. As explained further below, the steering element 164 may provide an actuator for selectively curving, bending, or otherwise steering a distal portion 115 of the catheter 112. Also as explained further below, the steering adjustment member 180 may provide a mechanism for varying the steerability of the distal portion 115, e.g., allowing a user to control a radius of curvature and/or how much length of the distal portion 115 is steered.

The catheter 112 may also include other components similar to other embodiments described herein, e.g., a handle (not shown) on the proximal end, a balloon or other expandable member (also not shown) on the distal end 16, and/or an imaging assembly (also not shown) for imaging through the balloon or otherwise beyond the distal end 116 of the catheter 112. Optionally, the catheter 112 may include one or more other components, e.g., a syringe or other source of inflation media, a monitor or other output device, a guidewire, a needle, a guide catheter, an energy probe, and the like (not shown), similar to the previous embodiments.

With additional reference to FIGS. 16A and 16B, the catheter 112 may be substantially flexible, semirigid, and/or rigid along its length, and may be formed from a variety of materials, including plastic, metal, and/or composite materials, similar to the previous embodiments. For example, as shown, the catheter 12 may include a substantially flexible distal portion 115 that may be steerable or bendable. As used herein, "bending" or "bendable" may refer to the ability of one or more portions of the catheter 112 to curve or otherwise bend in a controlled and predictable manner, e.g., without buckling or kinking.

Returning to FIGS. 14 and 15, the lumen 120 generally includes a first region 120a that extends substantially parallel to a center of modulus 184 of the distal portion 115 and a second region 120b offset from the center of modulus 184. Thus, the lumen 120 may have a predetermined "keyhole" cross-section that intersects the center of modulus 184 of the distal portion 115, and includes one or more regions offset radially from the center of modulus 184. For example, the first and second regions 120a, 120b may only partially overlap, thereby creating a ridge or partition 120c between the first and second regions 120a, 120b. Optionally, the first and second regions 120a, 120b may have similar or different shapes and/or sizes, e.g., depending upon the size and/or cross-section of the steering element 164 and/or steering adjustment member 180, as explained further below.

As used herein, "center of modulus" refers to a location within the catheter 112 (e.g., at least within the distal portion 115), that, if a tensile or compressive force is applied axially at the location, a bending moment will not be created. Stated differently, if an axial force is applied along the center of modulus 184, the distal portion 115 of the catheter 112 may not bend, while, if an axial force is applied parallel to but radially offset from the center of modulus 184, the distal portion 115 may bend due to the resulting bending moment, as described further below. If the construction of a catheter is substantially symmetrical about a central longitudinal axis of the catheter, the center of modulus may coincide with the central longitudinal axis. If, however, the cross-section of the catheter is not substantially symmetrical, e.g., due to different materials around the catheter and/or locations of lumens or components that may affect local stiffness, the center of modulus may be offset radially outwardly from the central longitudinal axis.

With continued reference to FIGS. 14 and 15, the catheter 112 includes a pullwire or other steering element 164 that is slidably disposed within the lumen 120. For example, the steering element 164 may extend from the proximal end of the catheter 112 through the distal portion 115 to the distal end 116. The steering element 164 may be fixed at the distal end 116, e.g., radially offset from the center of modulus 184. The steering element 164 may have sufficient flexibility to curve or otherwise bend as the catheter 112 is bent. In addition, the steering element 164 may have sufficient tensile and/or column strength to be pulled and pushed axially within the lumen 120, e.g., from the proximal end of the catheter 112, without substantial deformation or other failure.

In one embodiment, the steering element 164 may be an optical fiber, e.g., part of an imaging system carried by the catheter 112. For example, as shown in FIGS. 2 and 4A-4D, optical imaging fiber 64 (used to transfer images from the distal end 16 of the catheter 12 to the proximal end 14) may be used as a steering element. In this embodiment, the optical fiber 64 may be fixed to the objective lens 66 (not shown, see, e.g., FIG. 7), which may be mounted to the distal end 16 of the catheter 12. The objective lens 66 may be radially offset from the center of modulus (not shown) to allow for bending of the distal portion 115.

In addition or alternatively, an illumination fiber 62 may be used as a steering element (not shown). In another alternative, the steering element 164 may be a dedicated pullwire, e.g., formed from metal, such as stainless steel or Nitinol, plastic, or composite material, having a round, polygonal, or other cross-section. In other alternatives, multiple steering elements (not shown) may be provided, e.g., with a single or multiple steering adjustment members (also not shown), that may allow steering in multiple directions and/or multiple portions of the catheter.

Returning to FIG. 14, the steering element 164 may have a cross-section smaller than the first and second regions 120a, 120b to allow the steering element 164 to pass between the first and second regions 120a, 120b. The steering element 164 and/or the wall of the lumen 120 may be coated, e.g., to reduce friction between the steering element 164 and the wall of the lumen 120. In addition or alternatively, all or a portion of the steering element 164 may be provided in a relatively thin sheath or sleeve, e.g., a tube of PET, polyimide, or other polymer, to enhance lubricity, distribute forces, reduce abrasion, and/or otherwise protect the steering element 164.

With continued reference to FIGS. 14 and 15, the steering adjustment member 180 may be an elongate member slidably disposed within the lumen 120 for selectively directing a portion of the steering element 164 between the first and second regions 120a, 120b, e.g.,

to change a bending moment and/or bendable length of the distal portion 115.

For example, the steering adjustment member 180 may be an elongate rod, tube, wire, or other guide slidably received in the lumen 120. Similar to the steering element 164, the steering adjustment member 180 may be sufficiently flexible not to substantially interfere with bending of the distal portion 115 (or other portion of the catheter 112 through which the steering adjustment member 180 passes). In addition, the steering adjustment member 180 may have sufficient tensile and/or column strength to allow the steering adjustment member 180 to be pushed and pulled within the catheter 112, e.g., from the proximal end.

The steering adjustment member 180 may terminate in a rounded, tapered, and/or other substantially atraumatic distal tip 182. Optionally, the distal tip 182 may be polished, coated, or otherwise treated to reduce friction and/or otherwise facilitate sliding the distal tip 182 along the steering element 164 without abrading or otherwise damaging the catheter body and/or steering element 164, as described below.

As best seen in FIG. 15, the steering adjustment member 180 may be slidably received in the second region 120b of the lumen 120. For example, the steering adjustment member 180 may have a substantially round cross-section only slightly smaller than the second region 120b, but having a diameter larger than a width of the partition 120c. Thus, the steering adjustment member 180 may be slidable within the second region 120b, but may not pass through the partition 120c into the first region 120a.

The steering adjustment member 180 may have a substantially uniform cross-section from the distal tip 182 proximally to the proximal end of the catheter 112. This may allow the steering adjustment member 180 to slide but be constrained within the lumen 120. Alternatively, the steering adjustment member 180 may have different cross-sections and/or materials along its length. For example, a distal portion of the steering adjustment member 180 extending through the distal portion 115 of the catheter 112 may have the cross-section shown in FIG. 15, while a proximal portion (not shown) of the steering adjustment member 180 may have a different cross-section or material, e.g., a smaller wire that may be coupled to the portion within the distal portion 115 of the catheter 112. For example, materials and sizes may be selected to minimize any impact the steering adjustment member 180 has on the flexibility or other properties of the catheter 112.

Turning to FIGS. 16A and 16B, during use, the distal end 116 of the catheter 112 may be inserted into a body lumen, e.g., until the distal portion 115 is received within a heart chamber or other body cavity, similar to the previous embodiments. With the distal tip 182 of the steering adjustment member 180 disposed within the catheter 112 as shown in FIG. 16A, the steering element 164 may be pulled, causing the distal portion 115 to bend as shown. With additional reference to FIGS. 14 and 15, the steering adjustment member 180 may constrain the steering element 164 within the first region 120a proximal to the distal tip 182, i.e., in region "A" shown in FIGS. 14 and 16A. Consequently, the portion of the steering element 164 located in region "A" is substantially aligned with the center of modulus 184 and is not subjected to a bending moment when the steering element 164 is pulled.

In contrast, the portion of the steering element 164 beyond the distal tip 182 of the steering adjustment member 180 , i.e., in region "B" shown in FIGS. 14 and 16A, is not constrained within the first region 120a. Thus, when the steering element 164 is pulled, the steering element 164 is free to pass into the second region 120b of the lumen 120, as best seen in FIG. 14, thereby creating a bending moment on the catheter 112 in region "B." This bending moment causes the distal portion 115 in region "B" to bend, as shown in FIG. 16A.

Turning to FIG. 16B, the steering adjustment member 180 has been partially withdrawn within the distal portion 115 of the catheter 112. Thus, the catheter 112 is now divided into a region "A'" proximal to the distal tip 182 and a region "B'" distal to the distal tip 182. As shown, a longer length of the distal portion 115 is now disposed beyond the distal tip 182 of the steering adjustment member 180, and may now be subject to a bending moment when the steering element 164 is pulled. Consequently, for a given proximal tensile force on the steering element 164, the distal portion 115 in FIG. 16B may be curved in a longer arc having a larger radius of curvature, as compared to FIG. 16A. Thus, the steering adjustment member 180 may change an initiation location (a location between regions "A" and "B") where the distal portion 115 of the catheter 112 is subject to bending, which may change the arc and/or radius of curvature of the distal portion 115 when the steering element 164 is subjected to a predetermined axial force.

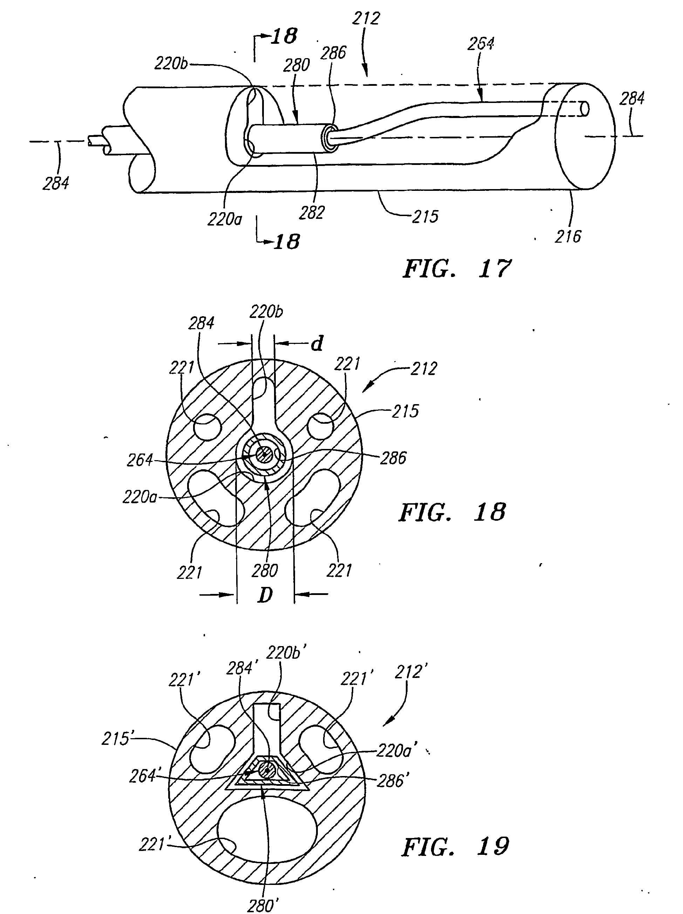

Turning to FIGS. 17 and 18, a distal portion 215 of another embodiment of a catheter 212 is shown that includes a steering element 264 and a steering adjustment member 280 disposed within a lumen 220, similar to the previous embodiments described herein. Optionally, the catheter 212 may include other components, similar to other embodiments described herein (not shown for simplicity). In addition, the catheter 212 may include one or more additional lumens 221, e.g., for inflation media, optical fibers, and/or accessories, also as described elsewhere herein.

At least the distal portion 215 of the catheter 212 includes a keyhole-shaped lumen 220 having a first region 220a aligned with a center of modulus 284 and a second region 220b radially offset from the center of modulus 284. As best seen in FIG. 18, the first region 220a is substantially round, thereby defining a diameter "D." The second region 220b extends from the first region 220a and has a width or cross-section "d" that is substantially smaller than the diameter "D," thereby providing the keyhole shape.

Similar to other embodiments herein, the steering element 264 may be slidably disposed in the lumen 220 and may be fixed to the catheter 212, e.g., at distal end 216, or otherwise distal to the distal portion 215. The steering element 264 may have a diameter or other cross-section at least slightly smaller than the width "d" of the second region 220b, such that a portion of the steering element 264 may pass from the first region 220a into the second region 220b, as shown in FIG. 17. The steering element 164 may be an optical fiber or other pullwire, similar to the previous embodiments.

The steering adjustment member 280 may also be slidably disposed within the lumen 220, e.g., within the first region 220a. As best seen in FIG. 17, the steering adjustment member 280 may be an elongate tubular member defining a passage 286 therein. The steering adjustment member 280 may have a diameter or other cross-section that is at least slightly smaller than "D" but larger than "d." Thus, the steering adjustment member 280 may be slidable axially within the first region 220a of the lumen 220 but may not enter the second region 220b.

The steering adjustment member 280 may be formed from a substantially flexible, semi-rigid, or substantially rigid material. For example, the steering adjustment member 280 may be formed from a length of tubing, e.g., stainless steel, PEEK, nylon, and the like. Optionally, a distal tip 282 of the steering adjustment member 280 may be radiused, polished, coated, and the like, similar to the previous embodiments, e.g., to reduce friction and/or abrasion between the steering element 264 and the steering adjustment member 280 or the catheter body.

The steering element 264 may pass through the lumen 286 within the steering adjustment member 280, yet allow the steering adjustment member 280 to move axially around the steering element 264. Optionally, the steering element 264 may be slidably received in a protective sleeve (not shown), and/or the steering element 264 and/or wall of the lumen 220 may be coated to reduce friction, similar to the previous embodiments. Thus, the steering adjustment member 280 may constrain the steering element 264 within the first region 220a proximal to a distal tip 282 of the steering adjustment member 280. The steering element 264 may remain unconstrained distal to the distal tip 282, e.g., such that the steering element 264 may enter the second region 220b of the lumen 220, e.g., when a proximal tension is applied to the steering element 264. With the steering element 264 free to enter the second region 220b, the region of the distal portion 215 beyond the distal tip 282 of the steering adjustment member 280 may be bent when an axial force is applied to the steering element 264, similar to the previous embodiments.

The steering adjustment member 280 may be directed distally, e.g., using an actuator (such as slider 36 shown in FIG. 5) on the proximal end of the catheter 212, to shorten the bendable region and/or decrease the radius of curvature. Conversely, the steering adjustment member 280 may be directed proximally to lengthen the bendable region and/or increase the radius of curvature when the steering element 264 is pulled. The distal tip 282 of the steering adjustment member 280 may provide a fulcrum initiation location for the catheter 212, e.g., a location beyond which the catheter 212 may be steered during use, e.g., as described above.

Turning to FIG. 19, still another embodiment of a catheter 212' is shown that includes a keyhole lumen 220' and optionally one or more additional lumens 221.' A steering adjustment member 280' is slidably disposed within the lumen 220' and a steering element 264' is also slidably disposed within the lumen 220,' within a lumen 286' extending through the steering adjustment member 280.' Similar to the previous embodiments, the lumen 220' includes a first region 220a' substantially aligned with a center of modulus 284' of the catheter 212' and a second region 220b' radially offset from the center of modulus 284.'

Unlike the previous embodiment, the first region 220a' has a frusto-conical shape, and the second region 220b' has a narrower shape extending radially from the first region 220a.' The steering adjustment member 280' is a tubular body, e.g., an elongate extrusion and the like, having a lumen 286' therein. The steering adjustment member 280' also has a frusto-conical shape (or optionally other shape, not shown) that is keyed to slide axially within the first region 220a' of the lumen 220' without crossing into the second region 220b.'

The steering element 264,' which may be similar to the optical fiber or other pullwires described herein, may be received through the lumen 286' of the steering adjustment member 280.' Thus, the steering element 264' within the lumen 286' may be constrained within the first region 220a,' i.e., substantially aligned with the center of modulus 284,' while the steering element 264' beyond a distal tip (not shown) of the steering adjustment member 280' may enter freely into the second region 220b.' Thus, the steering adjustment member 280' may be slid axially within the first region 220a' to move a fulcrum initiation location about which the catheter 212' may bend when the steering element 264' is subjected to axial force.

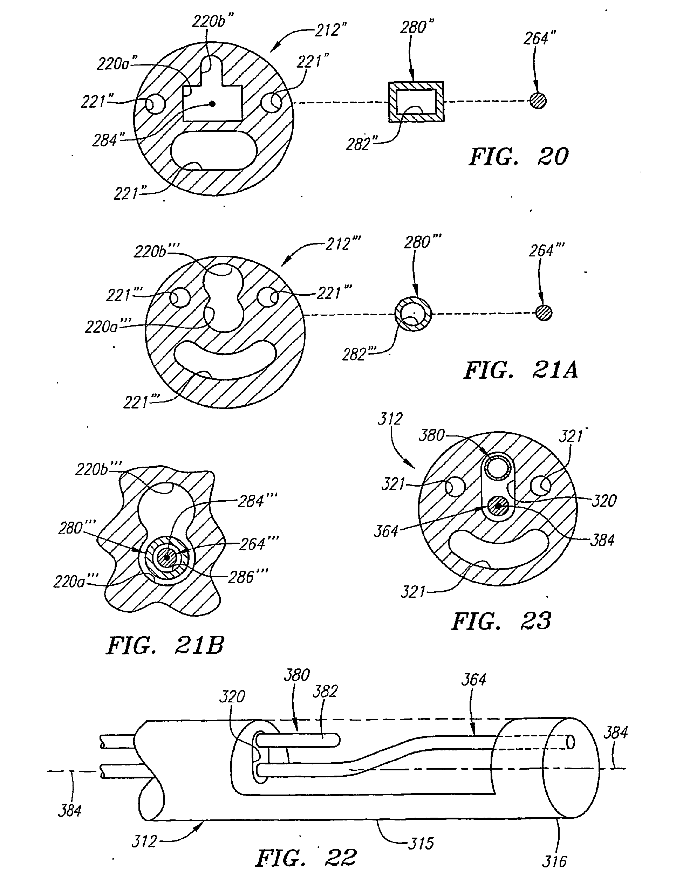

Turning to FIG. 20, yet another embodiment of a catheter 212" is shown that includes a keyhole lumen 220" and optionally one or more additional lumens 221." A steering adjustment member 280" is slidably disposed within the lumen 220" and a steering element 264" is also slidably disposed within the lumen 220," within a lumen 286" extending through the steering adjustment member 280." Similar to the previous embodiments, the lumen 220" includes a first region 220a" substantially aligned with a center of modulus 284" of the catheter 212" and a second region 220b" radially offset from the center of modulus 284."

Unlike the previous embodiment, the first region 220a' and the steering adjustment member 280" have a rectangular shape, and the second region 220b" has a narrower shape extending radially from the first region 220a." The steering adjustment member 280" may be keyed to slide axially within the first region 220a" of the lumen 220" without crossing into the second region 220b."

The steering element 264," which may be similar to the optical fiber or other pullwires described herein, may be received through the lumen 286" of the steering adjustment member 280." Thus, the steering element 264" within the lumen 286" may be constrained within the first region 220a," i.e., substantially aligned with the center of modulus 284," while the steering element 264" beyond a distal tip (not shown) of the steering adjustment member 280" may enter freely into the second region 220b." Thus, the steering adjustment member 280" may be slid axially within the first region 220a" to move a fulcrum initiation location about which the catheter 212" may bend when the steering element 264" is subjected to axial force.

Turning to FIGS. 21A and 21B, another alternative embodiment is shown of a catheter 212''' having a keyhole 220''' for receiving a steering element 264''' and steering adjustment member 280''' therein. A first region 220a''' of the lumen 220''' and the steering adjustment member 280''' may have corresponding circular cross-sections, allowing the steering adjustment member 280''' to slide axially within the first region 220a'''. A second region 220b''' of the lumen 220''' may have a circular cross-section larger than a diameter of the steering element 264''' such that, when the steering element 264''' enters the second region 220b''', the relatively large size of the second region 220b''' may reduce friction or other interference with the steering element 264.''.

Turning to FIGS. 22 and 23, a distal portion 315 of another embodiment of a catheter 312 is shown that includes a lumen 320 that at least partially intersects with a center of modulus 384 of the catheter 312. As best seen in FIG. 23, the lumen 320 may have an elliptical or other elongate cross-section that includes one side that intersects the center of modulus 384 and another side that is disposed radially outwardly from the center of modulus 384.

The catheter 312 includes a steering element 364 that is slidably disposed within the lumen 320 and is fixed to the distal end 316 of the catheter 312, i.e., distal to the steerable distal portion 315. The catheter 312 also includes a steering adjustment member 380 that includes a rounded distal tip 382 and is also slidably disposed within the lumen 320. As shown, the steering adjustment member 380 is disposed radially outwardly from the center of modulus 384, while the steering element 364 is disposed adjacent the steering adjustment member 380 along the center of modulus 384.

As best seen in FIG. 23, the steering adjustment member 380 may be a hollow body that may be at least partially filled with fluid, e.g., to cause the steering adjustment member 380 to expand within the lumen 320 and direct the steering element 364 towards the center of modulus. The steering adjustment member 380 may include a wire or other pusher element (not shown) allowing the steering adjustment member 380 to be pushed distally within the lumen 320 when the steering adjustment member is not fully expanded. Thus, the distal tip 382 of the steering adjustment member 380 may be moved axially, similar to the other embodiments herein, to adjust a fulcrum initiation location and thereby change a radius of curvature and/or arc length of the distal portion 315 of the catheter 312 that is bent when the steering element 364 is pulled.

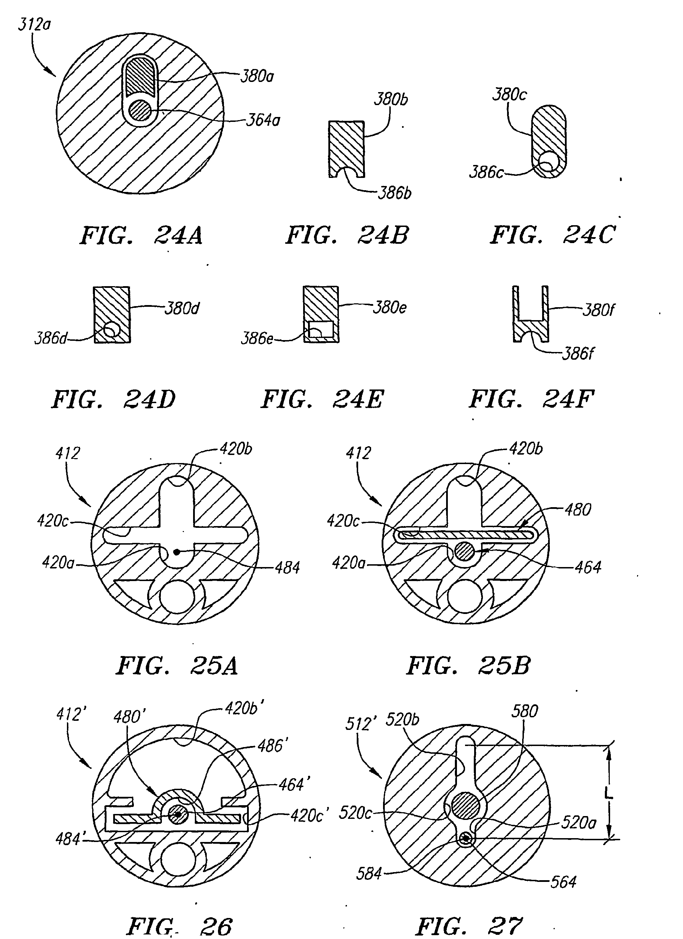

Alternatively, the steering adjustment member 380 may be an elongate wire (not shown), similar to the previous embodiments. In other alternatives, the steering adjustment member 380 may include elongate extrusions or other bodies having a variety of cross-sections. For example, FIG. 24A includes an embodiment of a steering adjustment member 380a having a concave inner surface that slides along the steering element 264a and a convex outer surface that slides along the wall of the lumen 320a. FIG. 24B includes an embodiment of a steering adjustment member 380b having a substantially rectangular cross-section other than a concave inner surface 386b that slides along the steering element (not shown).

FIG. 24C shows an embodiment of a steering adjustment member 380c having an elliptical cross-section and including a lumen 386c for receiving the steering element (not shown) therein. FIGS. 24D and 24E show a steering adjustment member 380d, 380e having a substantially rectangular cross-section and having a circular lumen 386d or rectangular lumen 386e for receiving the steering element (not shown) therein.

FIG. 24F shows an embodiment of a steering adjustment member 380f including a concave inner surface 386f that slides along the steering element (not shown) within a lumen of the catheter (also not shown). Unlike the previous embodiments, the steering adjustment member 3 80f has a portion of material removed, which may reduce a stiffness of the steering adjustment member 380f. Thus, the size and/or shape of the steering adjustment member may be selected to minimize any impact the steering adjustment member may have on flexibility or other properties of the catheter.

Turning to FIGS. 25A and 25B, another embodiment of a catheter 412 is shown that includes a keyhole lumen 420, a steering element 464, and a steering adjustment member 380, which may be generally constructed and/or used similar to other embodiments described herein. Similar to previous embodiments, the lumen 420 includes a first region 420a substantially aligned with a center of modulus 484 and a second region 420b offset radially outwardly from the center of modulus 484.

Unlike previous embodiments, the lumen 420 includes an elongated slotted region 420c that intersects between the first and second regions 420a, 420b. The steering adjustment member 480 may be an elongated, substantially flat body that may be slidably received in the slotted region 420c, thereby separating the first and second regions 420a, 420b from one another. The steering element 464, e.g., any of the embodiments described herein, may be received in the first region 420a, and the steering adjustment member 480 may be advanced into the catheter 412 to constrain the steering element 464 within the first region 420a.

Beyond the steering adjustment member 480, the steering element 464 may be free to cross the slotted region 420c into the second region 420b, thereby allowing the catheter 412 to bend beyond the steering adjustment member 480, similar to the previous embodiments. One advantage of the flat steering adjustment member 480 is the high moment of inertia the steering adjustment member 480 provides in a direction parallel to the slotted region 420c as compared to a direction perpendicular to the slotted region 420c. This relatively high moment of inertia may cause the catheter 412 to bend preferentially in a plane perpendicular to the slotted region 420c, e.g., in a plane within which the first and second regions 420a, 420b substantially lie. Thus, the steering adjustment member 480 may provide a hinge biasing the catheter 412 in a desirable manner.

Turning to FIG. 26, a cross-section of still another embodiment of a catheter 412' is shown that includes a lumen 420' within which a steering element 464' and steering adjustment member 480' may be slidably disposed. Similar to the previous embodiment, the lumen 420' may include a slotted region 420c' that receives the steering adjustment member 480' therein. Unlike the previous embodiment, the steering adjustment member 480' may include a recess 486' for constraining the steering element 464' along the center of modulus 484.'

The lumen 420' may also include a second region 420b' disposed radially outwardly from the center of modulus 484' within which the steering element 464' may pass beyond the steering adjustment member 380.' This embodiment may also bias the catheter 412' to bend in a predetermined plane, e.g., substantially perpendicular to the slotted region 420c.' In addition, this embodiment may minimize catheter material, thereby enhancing bending of the catheter 412' when the steering element 464' is pulled.

Turning to FIG. 27, another embodiment of a catheter 512 is shown that also includes a lumen 520, and a steering element 564 and steering adjustment member 580 slidably disposed therein. In this embodiment, the steering adjustment member 580 is slidably disposed in an enlarged region 520c of the lumen 520, separating a first region 520a aligned with a center of modulus 584 and a second region 520b disposed radially outwardly from the center of modulus 584. This profile may minimize a change in flexibility of the catheter 512 as compared to a catheter without the lumen 520. In addition, this configuration may maximize a lever arm, e.g., a distance "L" from the center of modulus 584 in the first region 520a to an outer portion of the second region 520b, which may enhance bending of the catheter 512.

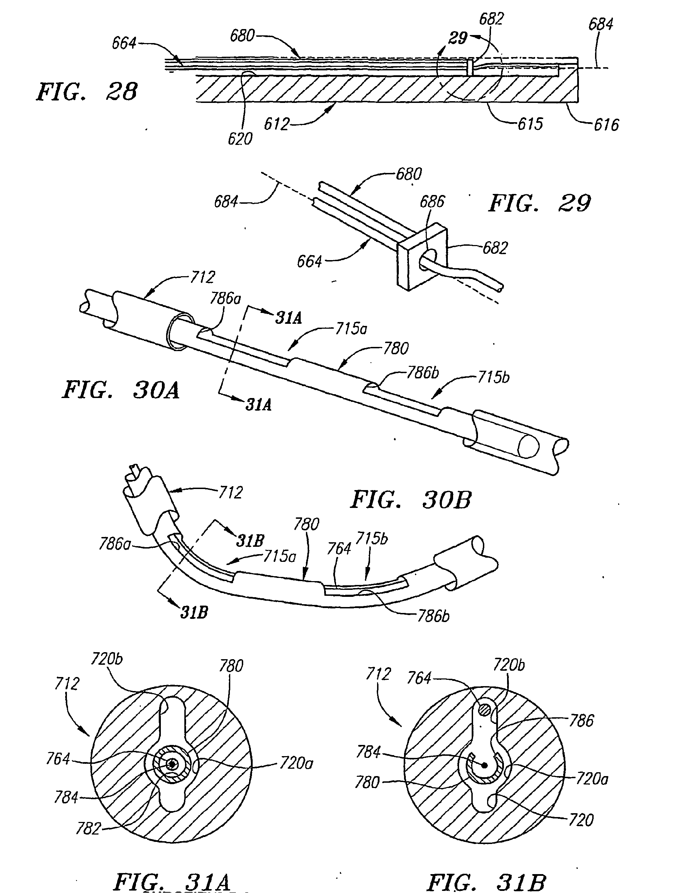

Turning to FIGS. 28 and 29, another embodiment of a distal portion 615 of a catheter 612 is shown that may be generally constructed and/or include similar to the other embodiments described herein. Generally, the catheter 612 includes a lumen 620 extending at least partially between a proximal end (not shown) and a distal end 616 of the catheter 612. Similar to the previous embodiments, the lumen 620 may intersect with a center of modulus 684 of the catheter 612, e.g., at least along the distal portion 615.

A steering element 664, e.g., an optical fiber or other pullwire, may be disposed within the lumen 620 that is slidable through the distal portion 615. The steering element 664 may be fixed on or adjacent the distal end 616, e.g., offset radially from the center of modulus 684. A steering adjustment member 680 is also slidably disposed within the lumen 620, e.g., adjacent the steering element 664.

The steering adjustment member 680 may include a distal tip 682 including an opening 686 therethrough that is substantially aligned with the center of modulus 684. As shown, the distal tip 682 may include a flange, block, or other structure coupled to the steering adjustment member 680 and having a size for sliding axially within the lumen 620 without substantially lateral movement. The steering element 664 may be slidably received through the opening 686 such that the steering element 664 proximal to the distal tip 682 is substantially aligned with the center of modulus 684, while the steering element 664 distal to the distal tip 682 may extend radially outwardly towards the fixation location on the distal end 616.

During use, the steering adjustment member 680 may be directed axially within the lumen 620 towards or away from the distal end 616 of the catheter 612. This action may shorten or lengthen the steering element 664 beyond the distal tip 682, which may provide a fulcrum initiation location beyond which the distal portion 615 may bend when an axial force is applied to the steering element 664. As the distal tip 682 is moved distally, the radius of curvature and/or arc length of the distal portion 615 may be reduced. As the distal tip 682 is moved proximally, the radius of curvature and/or arc length of the distal portion may be increased when the distal portion 615 is bent.

Thus, similar to the other embodiments described herein, the location of the distal end 616 of the catheter 612 may be adjusted in at least two ways. For example, adjusting the steering adjustment member 680 may change a radius of curvature of the distal portion 615. In addition, the size of the force applied to the steering element 664 may be proportional to the extent that the distal portion curves about the radius of curvature set by the steering adjustment member 680. Thus, as the steering element 664 is pulled using a greater force, the angle defined by the distal portion 615 from a longitudinal axis proximal to the distal portion 615 to the distal tip 616 may by varied, e.g., from zero to one hundred eighty degrees (0-180°), for each available radius of curvature allowed by the steering adjustment member 680.

Turning to FIGS. 30A and 30B, another embodiment of a catheter 712 is shown that includes multiple steerable regions 715a, 715b. It will be appreciated that any number of steerable regions may be provided, and the two steerable regions shown are merely exemplary. With additional reference to FIG. 31, the catheter 712 may include a keyhole lumen 720 therein that extends at least through the steerable regions 715, and may extend between proximal and distal ends (not shown) of the catheter 712. The catheter 712 may include any of the embodiments described elsewhere herein, e.g., including a handle, balloon, imaging assembly, and the like (not shown).

The catheter 712 includes a steering adjustment member 780 slidably disposed within the lumen 720, e.g., within first region 720a (shown in FIGS. 31A and 31B) and a steering element 764 also disposed within the lumen 720, e.g., at least partially within a lumen 782 of the steering adjustment member 780. As shown, in the steerable regions 715, a portion of the steering adjustment member 780 may be skived or otherwise partially removed to expose the steering element 764 within windows 786.

As shown in FIG. 31A, when the steering element 764 is constrained within the steering adjustment member 780, the steering element 764 may be substantially aligned with a center of modulus 784 of the catheter 712. Conversely, as in FIG. 31B, when the steering element 764 is disposed within windows 786, the steering element 764 may be free to enter an second region 720b of the lumen 720 radially offset from the center of modulus 784. Thus, when the steering element 764 is subjected to axial force, e.g., by pulling on a proximal end (not shown) of the steering element 764, the catheter 712 may bend along the steerable regions 715 as the steering element 764 enters the second region 720b of the lumen.

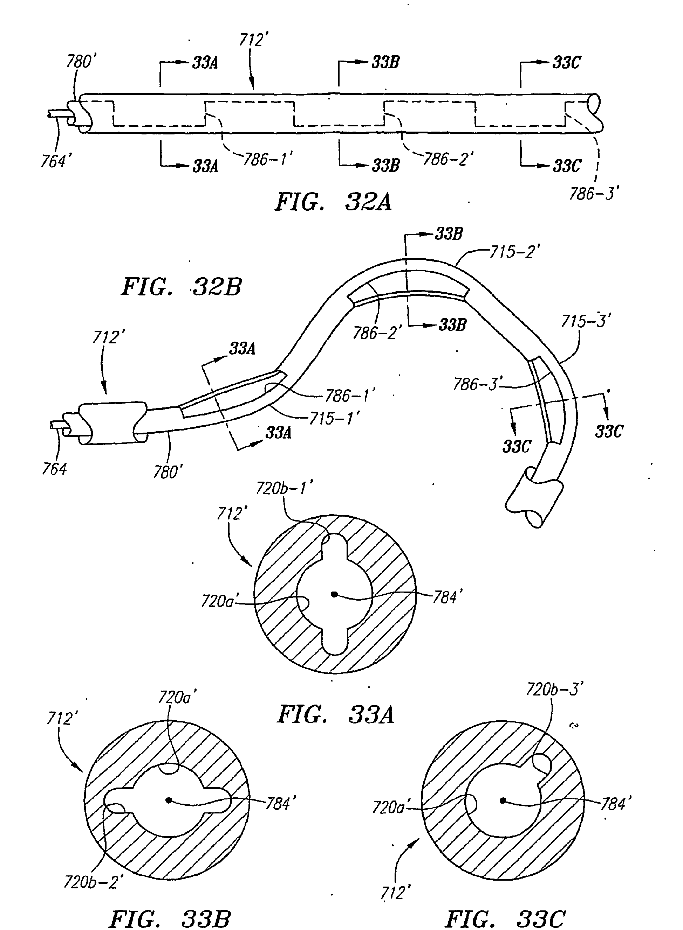

Alternatively, as shown in FIGS. 32A-33C, a catheter 712' may be provided with multiple steerable regions 715' that lie in one or more different planes. For example, as shown in FIGS. 33A-33B, the keyhole lumen 720' in the catheter 712' may include a first region 720a' substantially aligned with the center of modulus 784' and a second region 720b' in each of the steerable regions 715' that are angularly offset from one another.

As shown in FIGS. 33A and 33B, the second region 720b-1' in the first steerable region 715-1' may be offset by ninety degrees relative to the second region 720b-2' in the second steerable region 715-2.' Similarly, as shown in FIGS. 33B and 33C, the second region 720b-3' in the third steerable region 715-3' may be offset by forty five degrees relative to the second region 720b-2' in the second steerable region 715-2.' Thus, when the steering element 764' is subjected to an axial force, the steerable regions 715' may bend in different planes, as best seen in FIG. 32B.

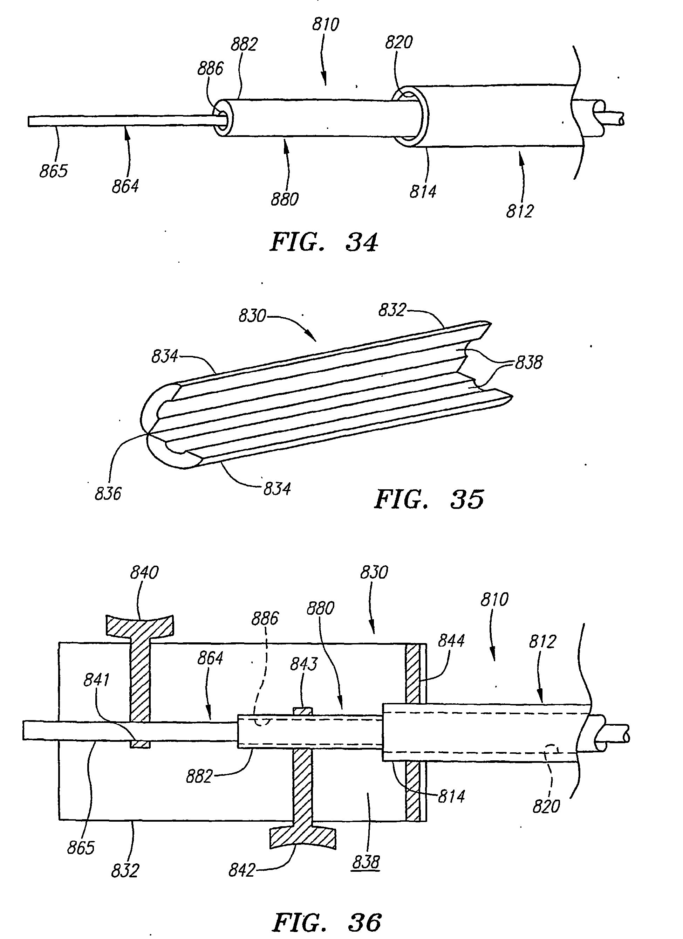

Turning to FIGS. 34-36, a proximal end 814 of an apparatus 810 is shown that includes a tubular member 812, a steering element 864, and a steering adjustment member 880. The tubular 812 may include any of the catheters described herein, e.g., including a balloon and/or imaging assembly on a distal end (not shown) of the tubular member 812. Alternatively, the tubular member 812 may be a guidewire or other relatively low profile device that may be introduced into a body lumen of a patient.

The tubular member 812 may include one or more lumens, e.g., lumen 820 for receiving the steering element 864 and/or steering adjustment member 880 therein. The lumen 820 may include a keyhole cross-section (not shown), e.g., at one or more locations within a distal portion of the tubular member 820, allowing steering similar to other embodiments described herein.

The steering adjustment member 880 may be an elongate tubular body including a proximal end 882, and a distal end disposed within a steerable distal portion (not shown) of the tubular member 812. The proximal end 882 of the steering adjustment member 880 may have a substantially round cross-section or other configurations corresponding to a similarly shaped region of the lumen 820. Further, a distal end of the steering adjustment member 880 may have a cross-section corresponding to in a region of the lumen 820 within the distal portion of the tubular member 812, thereby allowing the steering adjustment member 880 to slide within the distal portion, similar to other embodiments described herein.

The steering element 864 may be an elongate member, e.g., an optical fiber or pullwire that is slidably disposed within a lumen 886 of the steering adjustment member 880. A distal end (not shown) of the steering element 864 may be fixed, e.g., to the distal end of the tubular member 812 or other location distal to the steerable distal portion, also similar to other embodiments described herein.

A proximal end 865 of the steering element 864 may extend from the proximal end 882 of the steering adjustment member 880, and the proximal end 882 of the steering adjustment member 880 may extend from the proximal end 814 of the tubular member 812. Instead of providing a permanent handle fixed to the proximal end 814 of the tubular member 812 (such as the handle 30 shown in FIG. 2), a removable handle 830 may be provided (such as that shown in FIGS. 35 and 36). Thus, if it is desired to advance a catheter or other instrument (not shown) over the tubular member 812, the handle 830 may be removed, providing a relatively low profile proximal end of the apparatus 810 over which an instrument may be introduced.

Turning to FIG. 35, the handle 830 may include a clamshell housing 832 that may receive the proximal ends 814, 865, 882 of the apparatus 810 therein. As shown, the clamshell housing 832 may include two opposing portions 834 connected by a hinge 836 that may be mated together and locked, e.g., by cooperating detents, a latch, or other locking mechanism (not shown). The housing 832 may include a passage 838 between the opposing portions for receiving the proximal end of the apparatus 810, as shown in FIG. 36.

With particular reference to FIG. 36, the housing 832 may include one or more actuators 840, 842 for actuating or otherwise manipulating the steering element 864 and/or steering adjustment member 880. In addition, the housing 832 may include one or more shoulders or other elements 844 that may engage the proximal end 814 of the tubular member 812, e.g., to substantially fix the housing 832 relative to the tubular member 812. Optionally, the housing 832 and/or tubular member 812 may be secured to one another using one or more connectors or detents, an interference fit, complementary male and female components, and the like.

When the apparatus 810 is received in the housing 832, a first actuator 840 may substantially engage the proximal end 865 of the steering element 864, and a second actuator 842 may substantially engage the proximal end 882 of the steering adjustment member 880. For example, the first and second actuators 840, 842 may include hooks, pockets, or other connectors 841, 843 for receiving a portion of the proximal ends 865, 882 therein. The connectors 841, 843 may frictionally engage the proximal ends 865, 882 such that subsequent axial movement of actuators 840, 842 causes corresponding axial movement of the steering element 864 and steering adjustment member 880, respectively.

As shown, the first and second actuators 840, 842 may include sliders that may be manipulated by the user, e.g., by pressing with the user's thumb, to cause the first and second actuators 840, 842 to move proximally or distally, e.g., within a slot (not shown) in the housing 832. For example, the first actuator 840 may be directed from a neutral position, e.g., where the steering element 864 is free from external forces and the distal portion is substantially straight, to apply an axial tension on the steering element 864 to bend the distal portion. The first actuator 840 may be directed proximally to apply a proximal tension on the steering element 864 or distally to apply distal compression to the steering element 864, as is known for pullwires and other steering elements. In addition or alternatively, the second actuator 842 may be directed between a distal position, e.g., where the steering adjustment member 880 at least partially constrains the steering element 864 within the distal portion, and a proximal position, e.g., where at least a portion of the steering element 864 is free to move within the distal portion, e.g., away from a center of modulus of the distal portion, to allow bending, similar to other embodiments described herein.

When it is desired to remove the handle 830, the opposing portions 834 of the housing 832 may be opened, which may automatically disengage the actuators 840, 842 from the steering element 864 and steering adjustment member 880. Alternatively, it may be necessary for the user to engage and/or disengage the actuators 840, 842, e.g., when the housing 832 is open. With the actuators 840, 842 disengaged, the handle 830 may then be removed from the apparatus 810.

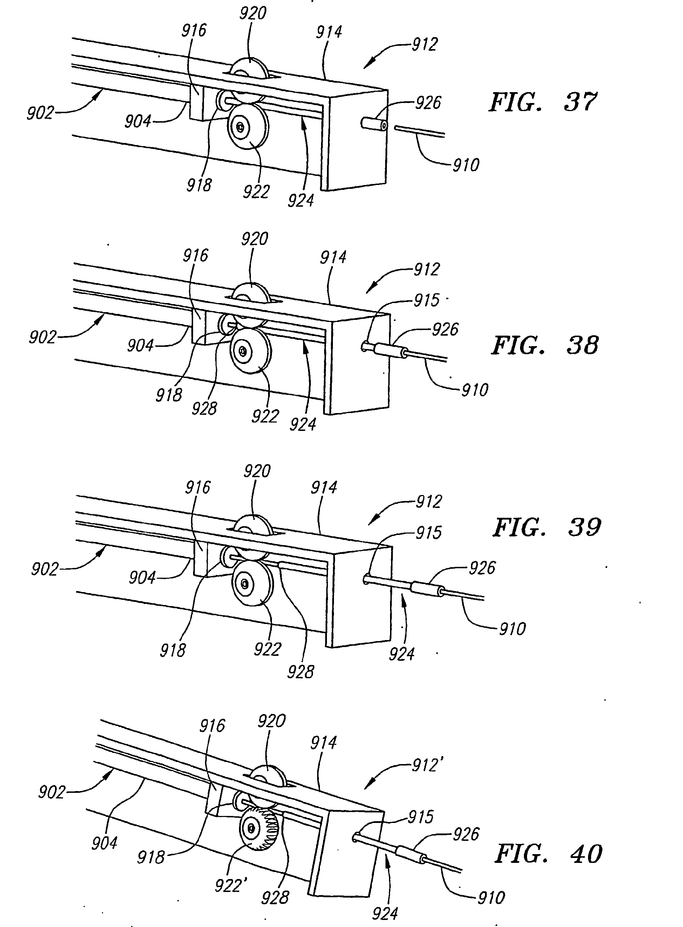

Turning to FIGS. 37-45, an apparatus 912 is shown that may be used for advancing, retracting, or otherwise manipulating a guidewire 910, which may be similar to the apparatus 810 described above or any of the other embodiments described herein. The apparatus 912 may facilitate manipulating a guidewire 910 using a single hand, e.g., simply by activating an actuator 920 on the apparatus 912. Thus, the user's other hand may be free to perform other tasks, such as manipulating a more distal portion (not shown) of the guidewire 910.

The apparatus 912 generally includes a housing 914 to which a proximal end 904 of a catheter 902 may be secured. The catheter 902 may be any tubular member, e.g., guide catheter, introducer sheath, or other instrument that may be introduced into a body lumen within a patient's body (not shown), e.g., using conventional procedures or any of the procedures described herein.

As shown in FIGS. 37-39, the housing 914 may include a mount 916 including one or more valves 918, e.g., a hemostatic valve. The proximal end 904 of the catheter 902 may be inserted into or otherwise secured to the mount 916, e.g., using one or more connectors (not shown), such that a lumen (not shown) of the catheter 902 is substantially aligned and/or sealed with the valve(s) 918 in the mount 916. Alternatively, one or more valves may be provided on the proximal 904 of the catheter 902 instead of on the mount 916. In this alternative, the mount 916 may include a passage (not shown) therethrough for receiving the proximal end 904 of the catheter 902.

The apparatus 910 also includes one or more drive members 920, 922 adjacent the mount 916 (and/or proximal end 904 of the catheter 902) for manipulating or otherwise actuating the guidewire 910 or other instrument received through the mount 916 into the catheter 902, as explained further below. As shown in FIG. 37, the apparatus 910 may include an upper drive member 920 and a lower drive member 922 that may define a passage therebetween for receiving the guidewire 910 therethrough. The drive members 920, 922 may include wheels rotatably mounted to the housing 814 by axles or other supports (not shown). Although the drive members 920, 922 may be spaced apart from one another, they may remain in frictional contact such that movement of the upper drive member 920 necessarily rotates the lower drive member 922 whether the guidewire 910 is present or not. In this configuration, the drive forces applied to the guidewire 910 when the upper drive member 920 is actuated may be essentially double compared to a single drive member.

Turning to FIGS. 37-39, the apparatus 912 also includes a valve passage slider 924 that may be removably inserted into the apparatus 912, e.g., for guiding the guidewire 910, as explained further below. The valve passage slider 924 includes a proximal end 926, e.g., exposed from the housing 914 through an opening 915, and a distal end 928 that is initially received through the valve(s) 918. The valve passage slider 924 may include a tubular body having a size for insertion through the valve(s) 918 and/or into the lumen of the catheter 902. The proximal end 926 may include an enlarged region or handle, which may facilitate manipulation of the valve passage slider 924, e.g., during removal from the housing 914, as explained further below.

When the valve passage slider 924 is in the position shown in FIG. 37, a guidewire 910 (including those with pre-shaped tips, such as "pigtails"), catheter (not shown, e.g. an infusion catheter, a guide catheter, and the like), or other instrument may be easily inserted into the proximal end 926 of the valve passage slider 024. Thus, the guidewire 910 may be advanced easily through the drive mechanism 920, 922, through the valve(s) 918, and into the lumen of the catheter 902.

During use, a guidewire 910 may be introduced into the proximal end 928 of the valve passage slider 924 and advanced until the guidewire 910 is received in the catheter 902. Turning to FIG. 38, with the guidewire 910 advanced, the valve passage slider 924 may be retracted from the valve(s) 918 yet still extend through the drive mechanism 920 ,922. In this position, the guidewire 910 may be advanced manually from outside the apparatus 910, e.g., using conventional manipulation, while benefiting from the valve(s) 918 that substantially isolate the lumen of the catheter 902 from the outside environment. The valve(s) 918 may be useful for various reasons, including preventing blood loss, facilitating infusion (e.g., of balloon infusate, therapeutic substances, and the like), and/or aspiration.

Turning to FIG. 39, the valve passage slider 924 may be removed further (and optionally entirely from the apparatus 910), exposing the guidewire 910 to the drive mechanism 920, 922. As shown, the guidewire 910 may be compressed between the drive members 920, 922 such that rotation of the upper drive member 920 drives the guidewire 910, e.g., distally into the catheter 902.

Turning to FIG. 40, an alternate embodiment of an apparatus 912' is shown that includes components similar to the previous embodiment (which are identified with the same reference numbers for convenience). The apparatus 912' includes a lower drive member 922' that includes a gear like drive member, e.g., including teeth or other elements designed to better grip the guidewire 910. For example, many guidewires may include one or more lubricious coatings (e.g., Glidewire™, and the like) that may otherwise impair engagement between the drive mechanism 920, 922.' In the embodiment shown, the gear-like drive member 922 may be in frictional contact with the upper drive member 920 such that rotation of the upper drive member 920 necessarily rotates the lower drive member 922.' This may increase the frictional forces applied to the guidewire 910, and thereby improve the efficiency of the drive mechanism 920, 922.'

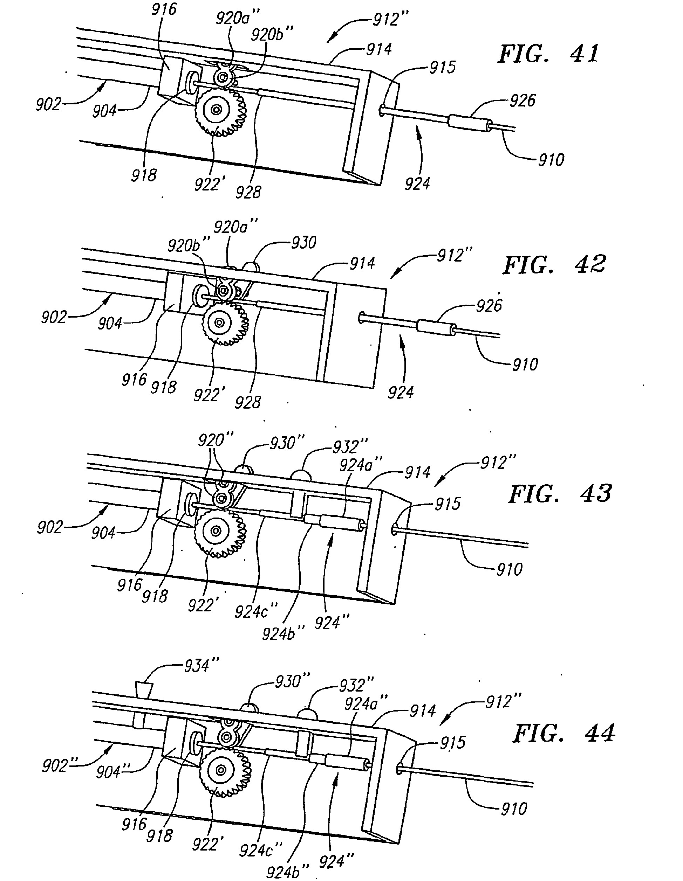

Turning to FIG. 41, another alternative embodiment of an apparatus 912" is shown that includes components similar to the previous embodiment (which are identified with the same reference numbers for convenience). Unlike the previous embodiments, the upper drive member 920 has been replaced by two smaller upper drive members 920." This configuration allows for the direction of rotation of the uppermost drive member 920a" (relative to the outer surface of the housing 914) to be in the same direction as the subsequent driving direction of the guidewire 910, which may provide more intuitive manipulation of the guidewire 910.

FIGS. 42-44 show variations of the apparatus 910" shown in FIG. 41. For example, in FIG. 42, a ratchet mechanism 930" has been provided on the housing 914 that may be selectively coupled to one of the upper drive members 920." The ratchet mechanism 930" may allow a user to select a direction of propagation desired for the apparatus 912" (i.e. distally or proximally), and then selectively rotate/slide the uppermost drive mechanism 920a" "back and forth," without needing to remove a finger/hand from the uppermost drive member 920a." Only motion in the direction allowed by the ratchet mechanism 930" may be transferred to the guidewire 910 to drive the guidewire in the selected direction, while motion in the opposite direction may do nothing (other than provide convenience to the user).

In FIG. 43, the valve passage slider 924" has been modified to include a telescoping mechanism (e.g., concentric tubes 924a"-924c" slidable relative to one another). The first tube 924a" may be fixed to the housing 914, e.g., adjacent opening 915, while the second and third tubes 924b," 924c" may be slidable, e.g., from an extended position (not shown), wherein at least the third tube 924c" extends through the drive mechanism 920," 922' and the valve(s) 918. The valve passage slider 924" also includes a slider or other actuator 932" slidably fixed to the housing 914 coupled to the third tube 924c."

By manipulating the actuator 932," the valve passage slider 924" may be retracted from the valve(s) 918 and/or drive mechanism 920," 922.' Thus, the valve passage slider 924" may be activated from a side of the apparatus 912" as opposed to the back of the apparatus 912." In this configuration, the valve passage slider 924" may also be advanced easily back through the drive mechanism 920," 922' and/or valve(s) 918 simply by advancing the actuator 932," which may increase convenience of the apparatus 912."

In FIG. 44, the apparatus 912" may also include a side port 934" provided in the housing 914 that communicates with the lumen of the catheter 902." The side port 934" may be located on the housing 914 to facilitate selective infusion into and/or aspiration from the lumen of the catheter 902."

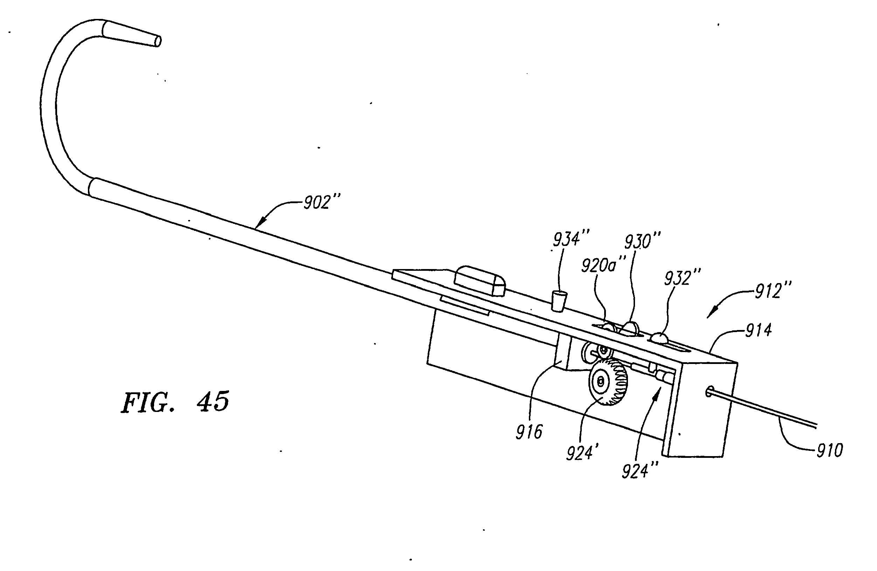

Turning to FIG. 45, an apparatus 912" similar to that shown in FIG. 44 is shown that includes a steerable catheter 902," which may be similar to any of the embodiments described herein. The apparatus 912" may facilitate rapid advancement and/or retraction of a guidewire 910 or other instrument, without losing the "feel" of a guidewire, which is often desired by users. The apparatus 912" may reduce the time that a user spends "hunting and pecking" for a hidden or difficult to access a target vessel, graft, or other body lumen, e.g., from another body lumen adjacent the target body lumen (not shown). Thus, the apparatus may be facilitate finding lumens with retrograde flow, or lumens that have been thrombosed or otherwise occluded. This apparatus 912" may also facilitate passing through venous valves in a retrograde fashion.

Optionally, other components may be added to any of the apparatus described above, such as apparatus 912 shown in FIG. 37 (used merely for convenience). For example, a motor (not shown) may be provided that is coupled to the drive mechanism. Mechanical advantage may be provided, e.g., to increase the push strength of the apparatus 912 while reducing the relative insertion distance of the guidewire 910 per rotation of the upper drive member 920. Mechanical disadvantage may be provided, e.g., to decrease the push strength of the apparatus 912, while increasing the relative insertion distance of the guidewire 910 per rotation of the upper drive member 920. In this alternative, mechanical disadvantage may heighten the responsive "feel" of the guidewire 910, which a physician may be used to encountering when inserting a guidewire. For example, any resistance due to an encountered obstacle in the body may be amplified backwards through the mechanical disadvantage to the user.

Other variations may include a variable mechanical advantage that would allow a user to locate a preferred "feel" or to otherwise increase or decrease the insertion distance of the guidewire per rotation of the upper drive member. Optionally, the apparatus 912 may allow the guidewire 910 to be rotated in addition to being advanced and/or retracted axially. It may be desirable to rotate a guidewire about its longitudinal axis in conjunction with advancement and/or retraction.

For example, in one embodiment, a wheel or other round driving mechanism (not shown) may be placed orthogonal to the guidewire 910 that may be "frictionally" and/or tangentially connected such that rotation of the wheel rotates the guidewire 910. Alternatively, the entire apparatus 910 may be rotatable about its longitudinal axis, e.g., relative to a stationary handle or other portion (not shown) extending from the back of the housing 914.

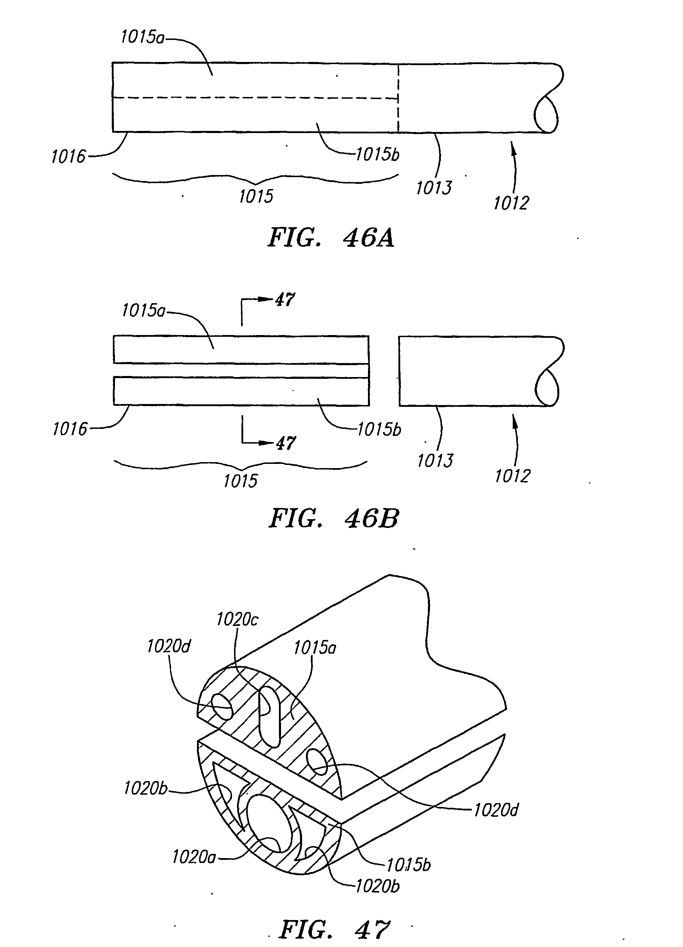

Turning to FIGS. 46A and 46B, another embodiment of a steerable catheter 1012 is shown that may include any of the components described in connection with any of the other embodiments described herein, e.g., a steering element, a steering adjustment member, a balloon, an imaging assembly, and the like (all not shown for simplicity). Generally, the catheter 1012 includes a proximal end (not shown), a distal end 1016 sized for insertion into a body lumen, and a steerable distal portion 1015.

As shown in FIG. 47, the catheter 1012 may include one or more lumens 1020, e.g., an accessory lumen 1020a, inflation lumens 1020b, an imaging fiber lumen 1020c, and illumination fiber lumens 1020d, similar to other embodiments described herein. The catheter 1012 may be manufactured to provide the lumens 1020 in a predetermined configuration that may enhance bending of the distal portion 1015 in a desired, predictable manner. For example, the lumens 1020b may be arranged in a symmetrical configuration substantially perpendicular to a bending plane of the distal portion 1015, e.g., a plane intersecting vertically through the cross-section shown in FIG. 47.

This symmetry may allow the distal portion 1015 to extend or compress more predictably during bending in the desired bending plane. For example, because illumination fibers (not shown) in the illumination fiber lumens 1020d may have relatively high modulus, a pair of illumination fiber lumens 1020d may be oriented substantially perpendicular to the desired bending plane. Because of their relatively high modulus, a pair of illumination fibers within the lumens 1020b may tend to bend in unison, i.e., substantially within to the bending plane. Otherwise, bending out of this plane would require one illumination fiber to be in compression while the other is in tension, which the fibers may tend to resist.

In addition or alternatively, to bias the distal portion 1015 to bend within the desired bending plane, the distal portion 1015 may be made from a composite structure that provides a hinge. A hinge mechanism may facilitate providing a single plane steerable catheter that substantially maintains deflection in a desired bending plane. In addition, a hinge mechanism may improve transmission of torque from the catheter body to a deflected tip, such as a balloon imaging catheter is used to scan or otherwise image along a heart wall, as described elsewhere herein.

Hinges may be constructed of a single element or multiple mechanical elements disposed within the catheter body. An effective hinge may also be constructed by creating a composite catheter body with varying modulus such that the catheter tends to bend predictably in a desired direction when subjected to internal or external forces. Such modulus may be a product of lumen configuration, material properties used, or other parameters (or a combination of such parameters).

For example, turning to FIG. 47, the catheter 1012 may include a composite distal portion 1015. An upper half or portion 1015a of the distal portion 1015 may be formed from a material having a first modulus, while a lower half or portion 1015b of the distal portion 1015 may have a second modulus greater than the first modulus. During bending, the lower modulus material of the upper portion 1015a may readily compress or stretch during bending compared to the higher modulus material of the lower portion 1015b. Consequently, this composite hinge may result in a bending plane that intersect through the upper and lower portions 1015a, 1015b, i.e., between the lower modulus material and the higher modulus material.

The upper and lower portions 1015a, 1015b may be bonded or otherwise attached to one another to provide the distal portion 1015. In addition, the composite distal portion 1015 may be attached to a proximal portion 1013 of the catheter 1012. For example, the proximal and distal portions 1013, 1015 may be butted together and bonded, fused by at least partially softening the ends, or otherwise attached. In addition or alternatively, the proximal and distal portions 1013, 1015 may partially overlap one another and/or may include one or more connectors (not shown). Optionally, a braid, sleeve, or other material (not shown) may be wrapped around, bonded, or otherwise provided to reinforce the junction between the proximal and distal portions 1013, 1015, which may also increase transmission of torque during bending.

Optionally, the catheter 1012 may include a resistive mechanism, e.g., within a handle on the proximal end (not shown).

The resistive mechanism may cause the distal portion 1015 to hold or otherwise maintain a shape that is set, e.g., a curve that is created when a steering element and/or steering adjustment member (both not shown) are manipulated or otherwise set in a desired manner. Exemplary resistive mechanisms are described elsewhere herein, e.g., in conjunction with FIGS. 6A and 6B.

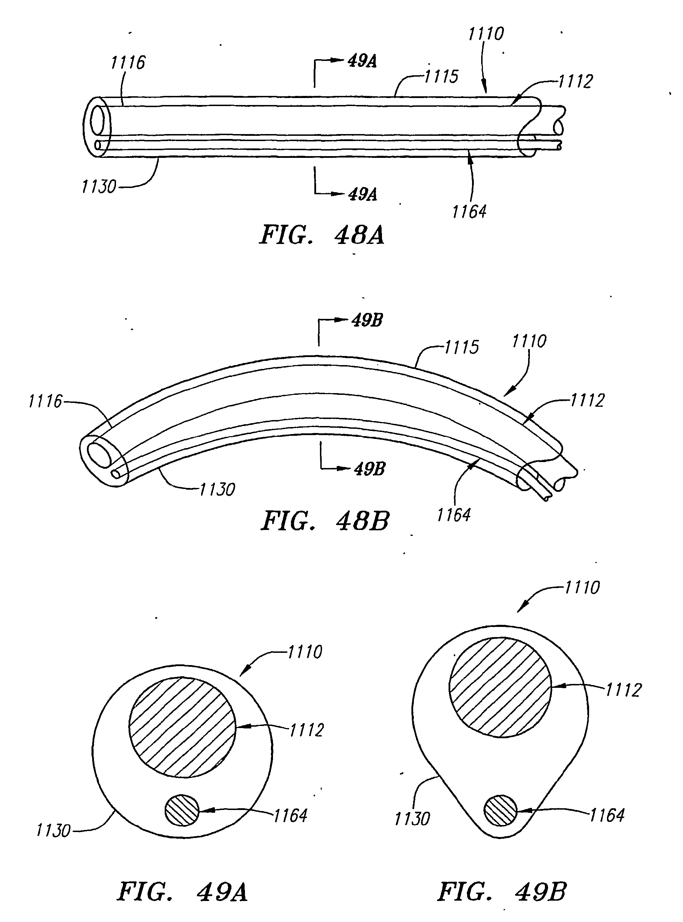

Turning to FIGS. 48A and 48B, another embodiment of a steerable catheter apparatus 1110 is shown that includes an external steering element 1164, instead of an internal steering element, as described elsewhere herein. The apparatus 1110 generally includes a catheter 1112, a steering element 1164, and an outer sleeve 1130 surrounding at least a steerable distal portion 1115 of the catheter 1112.

The catheter 1112 may be an elongate tubular member including a proximal end, a distal end 1116 insertable into a body lumen, and one or more lumens (not shown) extending therebetween, similar to other embodiments described herein. The steering element 1164 may be an optical fiber or other pullwire, also similar to other embodiments described herein. The steering element 1164 may be fixed or otherwise attached to the distal end 1116 of the catheter 1112, e.g., radially offset from a center of modulus (not shown) of the catheter 1112.

The steering element 1164 may be freely disposed adjacent an outer surface of the distal portion 1115 such that the steering element 1164 may move away from the catheter 1112, as described further below. The steering element 1164 may extend along an outer surface of the catheter 1112 all the way to the proximal end. Alternatively, the steering element 1164 may enter into a lumen (not shown) proximal to the distal portion 1115 that may slidably receive the steering element 1164 similar to other embodiments described herein. Thus, the steering element 1164 may be offset radially outwardly from a center of modulus along at least the distal portion 1115 of the catheter 1112, thereby creating a bending moment when the steering element 1164 is pulled that causes the distal portion 1115 to bend.

The sleeve 1130 may be a relatively thin-walled sheath, membrane, and the like, e.g., formed silicone, PET, or other elastomeric material. Thus, the sleeve 1130 may be substantially flexible, e.g., capable of expanding elastically away from the catheter 1112, yet resiliently biased to return against the catheter 1112. The sleeve 1130 may surround at least the distal portion 1115 of the catheter 1112 and the steering element 1164. Optionally, the sleeve 1130 may cover most or all of the catheter 1112 or may terminate proximal to the distal portion 1115, e.g., near a location where the steering element 1164 enters a lumen of the catheter 1112. The properties of the sleeve 1130 may be substantially uniform or variable along its length, if desired. Optionally, the sleeve 1130 may also be porous.

This arrangement of the steering element 1164 in combination with the sleeve 1130 may substantially reduce pull forces required to deflect the distal end 1116 of the catheter 1112. In addition or alternatively, this arrangement may effectively enable a variable cross-sectional geometry of the catheter 1112, which may change its bendability along the distal portion 1115 (or other steerable portions). For example, the moment created by the steering element 1164 may increase as it moves away from the outer surface of the catheter 1112 within the sleeve 1130.

For example, as shown in FIGS. 48A and 49A, when the steering element 1164 is substantially free from external forces, the sleeve 1130 may constrain the steering element 1164 against the catheter 1112. Although the distal portion 1115 may be flexible, the distal portion 115 is not subject to bending in this neutral position. Turning to FIGS. 48B and 49B, when the steering element 1164 is subjected to a tensile force or otherwise pulled, the distal portion 115 may bend. Because of the bending moment created, the steering element 1164 may be pulled away from the catheter 1112, thereby resiliently deforming the sleeve 1130. Thus, as best seen in FIG. 49B, the sleeve 1130 may be deformed into a composite shape, such as a tear-drop shape.

This shape change may increase the equivalent lever arm of the apparatus 1110, e.g., up to one hundred times, thereby significantly decreasing the pull force required to steer the distal portion 1115 in a desired manner. Optionally, to provide a discontinuous bending, one or more rings or other restraints (not shown) may be provided around the sleeve 1130. Optionally, the restraint(s) may be coupled to a steering adjustment member (not shown), similar to previous embodiments, thereby allowing the steerability of the catheter 1112 to be further adjusted.

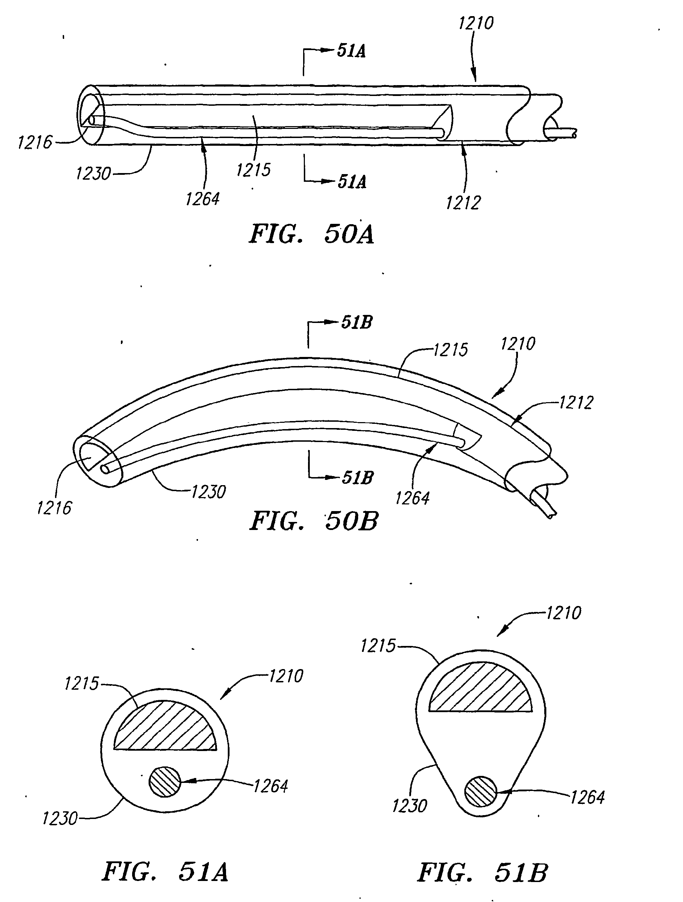

Turning to FIGS. 50A and 50B, an alternative embodiment of an apparatus 1210 is shown that includes a catheter 1212, steering element 1264, and flexible sleeve 1230, similar to the previous embodiment. Unlike the previous embodiment, a distal portion 1215 of the catheter 1212 may be modified to provide a hinge. For example, as best seen in FIGS. 51A and 51B, the distal portion 1215 may have a semi-circular or other partial cylindrical cross-section. Thus, the center of modulus of the distal portion 1215 may be offset radially away from the steering element 1264, thereby maximizing a lever arm created during bending.

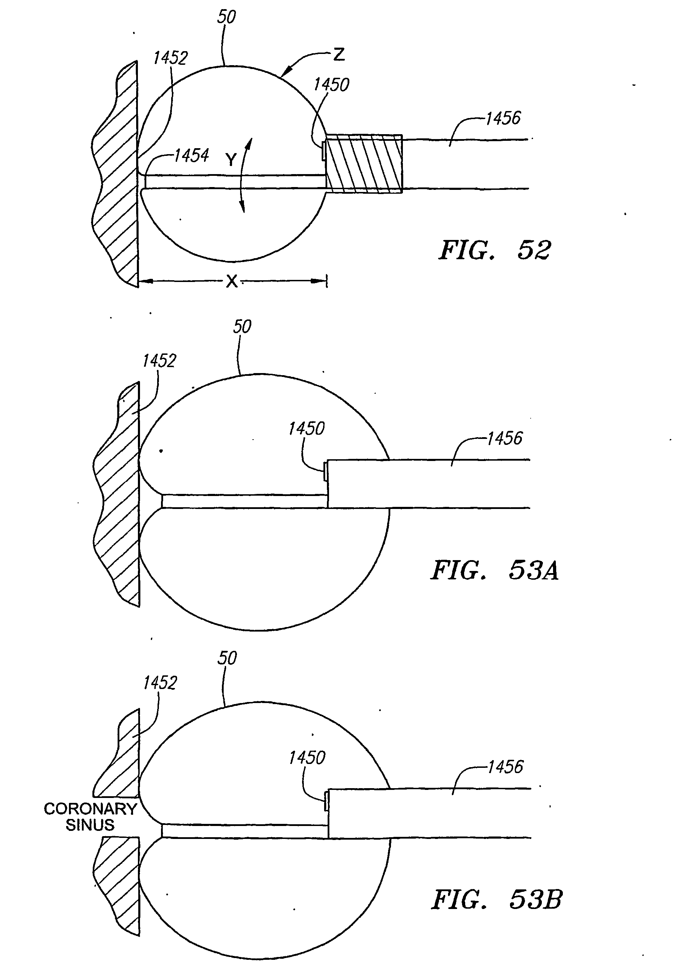

Turning to FIG. 52, an imaging apparatus is shown that may be similar to other embodiments described herein. FIG. 52 shows a transparent balloon 50 on a distal end of a catheter 1456 through which an imaging assembly may image. The view angle "y" of the imaging assembly is a function of a distal objective lens 1450 of the imaging assembly and the effective field of view is function of the distance "x" between the objective lens 1450 and the front face of the balloon 50. In one embodiment, the balloon face geometry may be optimized to appose to a circular region of tissue 1452 approximately equal to the effective field of view defined by "x" (the distance between the objective lens 1450 and the front face of the balloon 50) and the view angle "y." The distal end of the balloon 50 may be attached to the distal tip 1454 of the catheter 1456 in a manner optimized to minimize "puckering" or loss of apposition to tissue 1452, which may reduce visualization. In the case of imaging the coronary sinus or other vessel in a wall of an organ, e.g., the heart, the visual artifact created by "puckering" at the soft distal tip 1454 may look similar to a vessel, and create a false positive where the user mistakenly believes a target structure has been located.

The visual artifact created by this "puckering" of the balloon 50 may be seen in FIGS. 53A and 53B. In FIG. 53A, the image viewed due to the "puckering" of the balloon 50 is similar to the image viewed of the coronary sinus shown in FIG. 53B. In the embodiment shown, the proximal end of the balloon 50 may be attached at a location offset proximally from the distal end of the catheter 1456 to avoid the balloon 50 draping over the objective lens 1450 in its deflated or partially inflated state. By setting the bonding location proximate to the distal end of the catheter 1456, the risk of the balloon 50 at least partially obscuring the objective lens 1450 may be avoided or at least minimized throughout the full range of balloon 50 inflation.

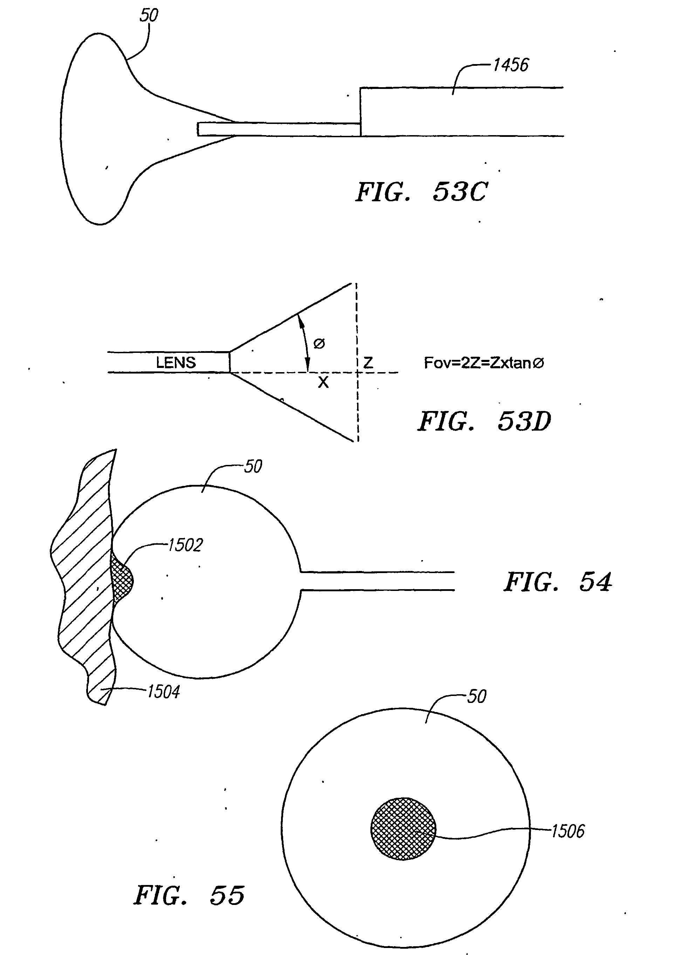

Turning to FIG. 53C, an embodiment of a balloon 50 is shown that has a shape that may increase the ability of the front face of the balloon 50 to displace blood from the field of view while minimizing balloon 50 volume. A smaller balloon 50 profile may reduce the propensity of blood flow to move the balloon 50. FIG. 53D illustrates a correlation between field of view and balloon 50 size. The field of view may be based on the following equation I:

$$\text{(I)} \qquad 2z = 2x \tan \Theta$$

Generally, the range of the field of view is within the range of about one and fifty millimeters (1-50 mm), or between about eight and eighteen millimeters (8-18 mm). In exemplary embodiments, the balloon 50 may have a diameter or other inflated size between about one and fifty millimeters (1-50 mm), or between about ten and twenty millimeters (10-20 mm).

In devices where balloons 50 or other expandable members are used to clear an optical path of blood such that anatomical features may be visualized, there is a possibility that blood may be trapped between the balloon (or other expandable member) and tissue. To reduce this from occurring, a balloon 50 on any of the catheter embodiments described herein may include an egress path 1500 to permit the optical clearing of a visual path outside the balloon 50 or expandable member. By clearing blood 1502 that may be trapped in the optical path, the catheter may be able to visualize or image anatomical features in an unobstructed manner.

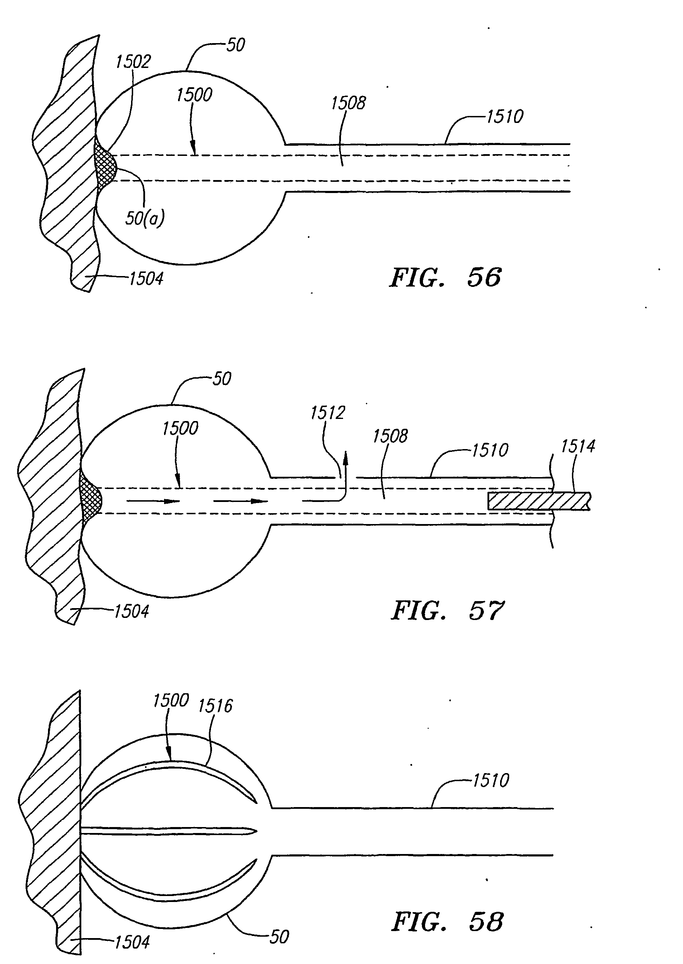

FIG. 54 illustrates a condition where blood 1502 is trapped between the surface of a balloon 50 and adjacent tissue 1504. If the balloon 50 "puckers," this may increase the chances that blood 1502 is trapped between the balloon 50 and adjacent tissue 1504. The trapped blood 1502 may appear in the visual field, e.g., creating an image as shown in FIG. 55 that includes a central opaque region 1506. In order to mitigate or eliminate the pooling of blood 1502 between the balloon 50 and tissue 1504, one or more egress paths 1500 may be provided that permit trapped blood 1502 to exit the optical path of the device. FIG. 56 illustrates one embodiment, wherein a centrally disposed lumen 1508 is provided in the balloon 50 as an egress path 1500.

As seen in FIG. 56, the centrally disposed lumen 1508 may originate at or near the distal-most tip 50 (a) (in the un-compressed, fully inflated state) of the balloon 50. The distal end of the lumen 1508 may be open to the external environment, thereby providing an entry path for the trapped blood 1502. The lumen 1508 may be disposed along the length of the balloon 50, e.g., communicating with a proximal elongate member 1510 which, in one embodiment, may be a flexible catheter and the like (not shown). The lumen 1508 may terminate at a proximal end (not shown in FIG. 56) of the catheter, thereby providing an outlet for the trapped blood 1502. In one aspect of the invention, the proximal end or outlet may terminate at the proximal end of the device outside the patient's body.

In one embodiment, the lumen 1508 may be sized to receive one or more instruments (not shown) therein. In this regard, the lumen 1508 may provide an accessory or working lumen in addition to functioning as the egress path 1500. Optionally, if desired, the interior of the lumen 1508 (or the instruments inserted therein) may be coated with a lubricious material to reduce frictional forces therebetween. If the lumen 1508 is sized to receive one or more instruments, the lumen 1508 may be sufficiently larger than the instruments, thereby providing a minimal amount clearance between the interior surface of the lumen 1508 and the exterior surface of the instrument to allow blood 1502 to egress around or along the length of the inserted instrument(s).

In an alternative configuration, as seen in FIG. 57, a side port 1512 may be provided proximal to the balloon 50 to serve as an outlet for the trapped blood 1502. In this embodiment, the side port 1512 may be located within the body (e.g., within a blood vessel) and permit trapped blood 1502 to egress from the trap to an exit location within the patient's body. The lumen 1508 may be located proximal to the side port 1512 and may be sealed, for example, by a moveable sealing member 1514 to avoid continuous leakage of blood outside the body.

Turning to FIG. 58, in another embodiment, one or more grooves 1516 may be provided on the external surface of the balloon 50 to aid in removing trapped blood 1502. The grooves 1516 may act as egress paths 1500 that permit blood 1502 to exit from the trap created between the balloon 50 and adjacent tissue 1504.

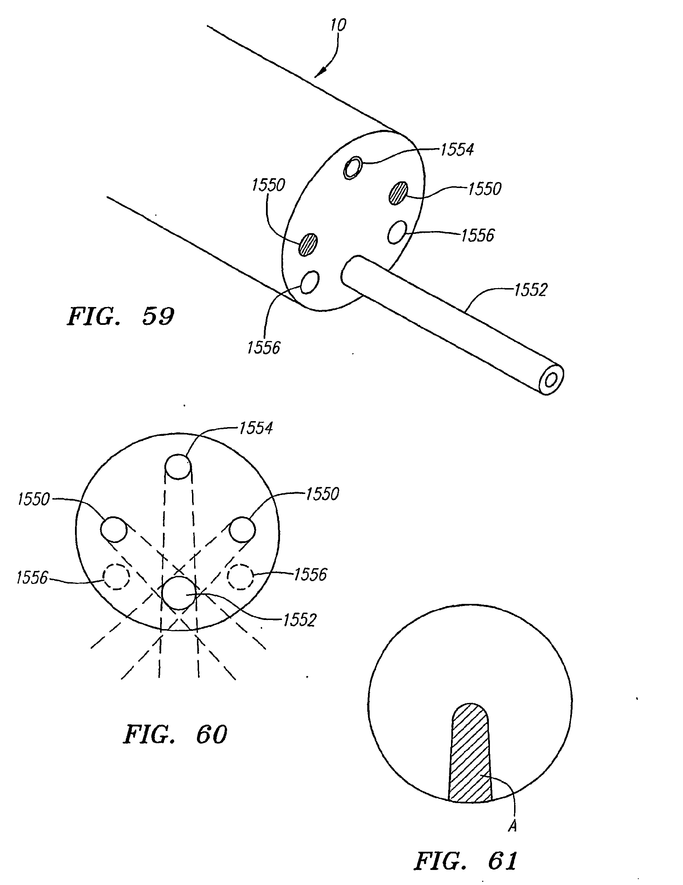

Turning to FIGS. 59 and 60, an exemplary configuration for illumination fibers 1550 of an imaging assembly is shown on the distal tip of the apparatus 10. The illumination fibers 1550 may be oriented to minimize the shadow cast by the tubular extension or balloon stabilization element 1552. As best seen in FIG. 59, the distal tip of the apparatus 10 includes an imaging fiber and/or lens 1554, a plurality of illumination fibers 1550 (two shown in FIG. 59), a plurality of balloon lumens 1556 (two shown in FIG. 59), and a balloon stabilization element 1552 (providing an extension of the accessory lumen). The illumination fibers 1550 may be oriented such that there are no dark areas (non-illuminated areas) except where the stabilization element blocks the field of view, as shown in FIG. 60.

FIG. 61 illustrates the resulting image with balloon stabilization element 1552 in the field of view. As seen in FIG. 61, the complete viewing area is illuminated except for a relatively narrow region where the balloon stabilization element 1552 casts a shadow that blocks the field of view (shown by shaded region A).

Additionally, the numerical aperture (sine of half-angle, as described above) of the illumination fibers 1550 may be optimized when the numerical aperture is similar to the numerical aperture of the imaging fiber 1554. Otherwise, a portion of the viewing plane may not be adequately illuminated. In one embodiment, the numerical aperture and corresponding area of illumination of each of the illumination fibers 1550 may be greater than the numerical aperture of the imaging fiber 1554. Thus, if both fibers 1550, 1554 terminate in substantially the same plane, e.g., at the face of the distal end of the catheter, the entire field of view of the imaging system may be adequately illuminated.

Additionally, the imaging and illumination components of the imaging assembly may be modified to utilize broader spectrum light, e.g., which may include light in the infrared (IR) spectrum. IR light may be used in a variety of settings to achieve imaging where visible light alone may be inadequate. The use of IR light to visualize in a blood filled environment has been described, for example, in Sedov, et al., *Vestnik Khirurgii* 157(1)68, 1998, and subsequently by Vadimovich, et al., *Heart Surgery Forum* 2(2):136, 1999.). IR light may be used in combination with visible spectrum imaging to image through blood and to distinguish by color.

These techniques may be used in temporal sequence or interspersed or side-by-side or otherwise combined. For example, IR imaging may be used initially to image through fluid, such as blood, to orient generally within a body cavity. Once the wall of the body cavity is being imaged, e.g., through a balloon 50, visible light imaging may be used, which may facilitate imaging tissue structures along the wall. This technique may be used with or without a balloon 50, and in the case in which the balloon 50 is utilized, the medium used to inflate the balloon 50 may be selected to minimize absorption of the particular light used for imaging.

It will be appreciated that elements or components shown with any embodiment herein are exemplary for the specific embodiment and may be used on or in combination with other embodiments disclosed herein.

While the invention is susceptible to various modifications, and alternative forms, specific examples thereof have been shown in the drawings and are herein described in detail. It should be understood, however, that the invention is not to be limited to the particular forms or methods disclosed, but to the contrary, the invention is to cover all modifications, equivalents and alternatives falling within the scope of the appended claims.

**Claims**

1.  An apparatus for introduction into a body lumen, comprising a tubular member (112, 212, 412) comprising a proximal end, a distal end (116, 216) sized for introduction into a body lumen, and a passage (120, 220, 420) extending along a steerable distal portion (115, 215) of the tubular member, the passage (120, 220, 420) comprising a first region (120a, 220a, 420a) and a second region (120b, 220b, 420b) offset from a center of modulus (184, 284, 484) of the distal portion (115, 215); and a steering element (164, 264, 464) slidably disposed through the passage (120, 220, 420) extending along the distal portion (115, 215), the steering element (164, 264, 464) comprising a proximal end disposed adjacent to the tubular member proximal end, and a distal end coupled to the tubular member distal end (116) beyond the distal portion (115, 215), **characterized by**:

    the first region (120a, 220a, 420a) being substantially aligned with the center of modulus (184, 284, 484) of the distal portion (115, 215); and

a steering adjustment member (180, 280, 480) slidably disposed within the passage (120, 220, 420) for selectively directing a portion of the steering element (164, 264, 464) between the first and second regions (120a, 220a, 420a, 120b, 220b, 420b), thereby changing a bending moment applied to the distal portion (115, 215) when an axial force is applied to the proximal end of the steering element (164, 264, 464).

2. The apparatus of claim 1, wherein the steering adjustment member comprises a guide (180, 280) slidable within one of the first and second regions (120a, 220a, 120b, 220b) for constraining a first portion of the steering element (164, 264) within the first region (120a, 220a) proximal to a distal tip (182, 282) of the guide (180, 280), while allowing a second portion of the steering element (164, 264) distal to the distal tip (182, 282) to enter the second region (120b, 220b).

3. The apparatus of claim 2, wherein the second region (220b) has a smaller cross-section than the first region (220a), the guide (280) having a cross-section larger than the second region (220b) that is slidable axially within the first region (220a).

4. The apparatus of claim 3, wherein the guide (480') comprises an arcuate cross-section defining a recess (486') for receiving a portion of the steering element (464') therein.

5. The apparatus of claim 2, wherein the guide (180) is slidable axially within the second region (120b), thereby blocking the steering element (164) from entering the second region (120b) proximal to a distal tip (182) of the guide.

6. The apparatus of claim 2, wherein the guide (280) is slidable axially within the first region (220a).

7. The apparatus of claim 1, wherein the passage (120, 220) comprises a keyhole cross-section defining the first and second regions (120a, 220a, 120b, 220b).

8. The apparatus of claim 1, wherein:

the steering element (164) is slidably disposed through the passage (120), a proximal portion of the steering element (164) being disposed within the first region (120a) of the passage (120) and a distal portion of the steering element (164) being disposed within the second region (120b) of the passage (120), and the distal end is coupled to the tubular member (112) within the second region (120b); and

the steering adjustment member (180) is slidably disposed within the second region (120b) of the passage (120) for selectively directing a portion of the steering element (164) between the first and second regions (120a, 120b), thereby changing a bending moment applied to the distal portion (115) when an axial force is applied to a proximal end of the steering element (164).

9. The apparatus of claim 8, wherein the passage (120) has a keyhole shaped cross-section.

10. The apparatus of claim 8, wherein the steering element (164) has a cross-section smaller than the first and second regions (120a, 120b).

11. The apparatus of claim 8, wherein the first and second regions (120a, 120b) overlap, thereby creating a ridge or partition (120c) between the first and second regions (120a, 120b).

12. The apparatus of claim 11, wherein the steering adjustment member (164) has a cross-section that is smaller than the second region (120b) but larger than a width of the partition (120c).

13. The apparatus of claim 1, wherein:

the steering element (264) is slidably disposed through the passage (220), a proximal portion of the steering element (264) being disposed within the fist region (220a) of the passage (220) and a distal portion of the steering element (264) being disposed within the second region (220b) of the passage (220), and the distal end is fixed to the tubular member (212) within the second region (220b); and

the steering adjustment member (280) is slidably disposed within the first region (212a) of the passage (212) for selectively directing a portion of the steering element (264) between the first and second regions (212a, 212b), thereby changing a bending moment applied to the distal portion (215) when an axial force is applied to a proximal end of the steering element (264).

14. The apparatus of claim 13, wherein the steering adjustment member (280) has a cross-sectional profile that is larger than the second region (212b) of the passage (212), thereby preventing the steering adjustment member (280) from entering the second region (212b) of the passage (212).

15. The apparatus of claim 13, wherein the steering element (264) is disposed within a lumen (286) formed in the steering adjustment member (280).

16. The apparatus of claim 13, wherein the passage (212) has a keyhole shaped cross-section.

17. The apparatus of claim 1, wherein:

the passage (420) of the tubular member (412) comprises an elongated slotted region (420c) intersecting the first and second regions (420a, 420b);

a proximal portion of the steering element (464) is disposed within the first region (420a) of the passage (420), a distal portion of the steering element (464) is disposed within the second region (420b) of the passage (420), and the distal end is fixed to the tubular member (412) within the second region (420b); and

the steering adjustment member (480) is slidably disposed within the elongated slotted region (420c) of the passage (420) for selectively directing a portion of the steering element (464) between the first and second regions (420a, 420b), thereby changing a bending moment applied to the distal portion when an axial force is applied to a proximal end of the steering element (464).

18. The apparatus of claim 17, wherein the steering adjustment member (480) comprises a substantially flat body that is slidably received in the slotted region (420c).

19. The apparatus of any preceding claim, further comprising an optical imaging assembly (60) carried by the distal end of the tubular member for imaging the tissue structure beyond the distal end, wherein the steering element comprises an optical fiber (62, 64) coupled to the imaging assembly (60).

20. The apparatus of claim 19, further comprising a substantially transparent expandable member (50) carried by the distal end of the tubular member, the expandable member (50) being expandable from a collapsed condition to an expanded condition, and at least partially surrounding the imaging assembly (60) for imaging beyond the distal end through the expandable member (50).

21. The apparatus of any preceding claim, wherein the steering element (164) comprises an optical imaging fiber (64).

22. The apparatus of any one of claims 1-20, wherein the steering element (164) comprises an illumination fiber (62).

**Patentansprüche**

1. Vorrichtung zum Einführen in ein Körperlumen, aufweisend ein röhrenförmiges Element (112, 212, 412), welches aufweist: ein proximales Ende, ein distales Ende (116, 216), das zum Einführen in ein Körperlumen bemessen ist, und einen Durchgang (120, 220, 420), der sich entlang einem steuerbaren distalen Abschnitt (115, 215) des röhrenförmigen Elements erstreckt, wobei der Durchgang (120, 220, 420) aufweist: einen ersten Bereich (120a, 220a, 420a) und einen zweiten Bereich (120b, 220b, 420b), der zu einer Modulmitte (184, 284, 484) des distalen Abschnitts (115, 215) versetzt ist, und ein Steuerelement (164, 264, 464), das gleitend durch den Durchgang (120, 220, 420) hindurch angeordnet ist, der sich entlang dem distalen Abschnitt (115, 215) erstreckt, wobei das Steuerelement (164, 264, 464) aufweist: ein proximales Ende, das benachbart zu dem proximalen Ende des röhrenförmigen Elements angeordnet ist, und ein distales Ende, das mit dem distalen Ende (116) des röhrenförmigen Elements über den distalen Abschnitt (115, 215) hinaus gekuppelt ist, **dadurch gekennzeichnet, dass:**

der erste Bereich (120a, 220a, 420a) im Wesentlichen mit der Modulmitte (184, 284, 484) des distalen Abschnitts (115, 215) fluchtet, und dass

ein Steuer-Anpassungs-Element (180, 280, 480) gleitend in dem Durchgang (120, 220, 420) zum selektiven Führen eines Abschnitts des Steuerelements (164, 264, 464) zwischen dem ersten und dem zweiten Bereich (120a, 220a, 420a, 120b, 220b, 420b) angeordnet ist, wodurch ein Biegemoment, das auf den distalen Abschnitt (115, 215) aufgebracht wird, wenn eine axiale Kraft auf das proximale Ende des Steuerelements (164, 264, 464) aufgebracht wird, geändert wird.

2. Vorrichtung gemäß Anspruch 1, wobei das Steuer-Anpassungs-Element eine Führung (180, 280) aufweist, die in einem von dem ersten und dem zweiten Bereich (120a, 220a, 120b, 220b) gleiten kann, zum Beschränken eines ersten Abschnitts des Steuerelements (164, 264) in dem ersten Bereich (120a, 220a) proximal zu einer distalen Spitze (182, 282) der Führung (180, 280), während gleichzeitig zugelassen wird, dass ein zweiter Abschnitt des Steuerelements (164, 264) distal zu der distalen Spitze (182, 282) in den zweiten Bereich (120b, 220b) eintritt.

3. Vorrichtung gemäß Anspruch 2, wobei der zweite Bereich (220b) einen kleineren Querschnitt als der erste Bereich (220a) aufweist, wobei die Führung (280), die in dem ersten Bereich (220a) axial gleiten kann, einen Querschnitt aufweist, der größer als der zweite Bereich (220b) ist.

**4.** Vorrichtung gemäß Anspruch 3, wobei die Führung (480') einen bogenförmigen Querschnitt aufweist, der eine Aussparung (486') zum Aufnehmen eines Abschnitts des Steuerelements (464') definiert.

**5.** Vorrichtung gemäß Anspruch 2, wobei die Führung (180) axial in dem zweiten Bereich (120b) gleiten kann, wodurch verhindert wird, dass das Steuerelement (164) in den zweiten Bereich (120b) proximal zu einer distalen Spitze (182) der Führung eintritt.

**6.** Vorrichtung gemäß Anspruch 2, wobei die Führung (280) axial in dem ersten Bereich (220a) gleiten kann.

**7.** Vorrichtung gemäß Anspruch 1, wobei der Durchgang (120, 220) einen Schlüsselloch-Querschnitt aufweist, der den ersten und den zweiten Bereich (120a, 220a, 120b, 220b) definiert.

**8.** Vorrichtung gemäß Anspruch 1, wobei:

das Steuerelement (164) gleitend durch den Durchgang (120) hindurch angeordnet ist, wobei ein proximaler Abschnitt des Steuerelements (164) in dem ersten Bereich (120a) des Durchgangs (120) angeordnet ist und ein distaler Abschnitt des Steuerelements (164) in dem zweiten Bereich (120b) des Durchgangs (120) angeordnet ist, und das distale Ende im zweiten Bereich (120b) mit dem röhrenförmigen Element (112) verbunden ist, und
das Steuer-Anpassungs-Element (180) gleitend in dem zweiten Bereich (120b) des Durchgangs (120) zum selektiven Führen eines Abschnitts des Steuerelements (164) zwischen dem ersten und dem zweiten Bereich (120a, 120b) angeordnet ist, wodurch ein Biegemoment, das auf den distalen Abschnitt (115) aufgebracht wird, wenn eine axiale Kraft auf ein proximales Ende des Steuerelements (164) aufgebracht wird, geändert wird.

**9.** Vorrichtung gemäß Anspruch 8, wobei der Durchgang (120) einen schlüssellochförmigen Querschnitt aufweist.

**10.** Vorrichtung gemäß Anspruch 8, wobei das Steuerelement (164) einen Querschnitt aufweist, der kleiner als der erste und der zweite Bereich (120a, 120b) ist.

**11.** Vorrichtung gemäß Anspruch 8, wobei der erste und der zweite Bereich (120a, 120b) überlappt sein können, wodurch eine Kante oder eine Abtrennung (120c) zwischen dem ersten und dem zweiten Bereich (120a, 120b) gebildet wird.

**12.** Vorrichtung gemäß Anspruch 11, wobei das Steuer-Anpassungs-Element (164) einen Querschnitt aufweist, der kleiner als der zweite Bereich (120b), jedoch größer als eine Breite der Abtrennung (120c) ist.

**13.** Vorrichtung gemäß Anspruch 1, wobei:

das Steuerelement (264) gleitend durch den Durchgang (220) hindurch angeordnet ist, wobei ein proximaler Abschnitt des Steuerelements (264) in dem ersten Bereich (220a) des Durchgangs (220) angeordnet ist und ein distaler Abschnitt des Steuerelements (264) in dem zweiten Bereich (220b) des Durchgangs (220) angeordnet ist, und das distale Ende in dem zweiten Bereich (220b) an dem röhrenförmigen Element (212) fixiert ist, und
das Steuer-Anpassungs-Element (280) gleitend in dem ersten Bereich (212a) des Durchgangs (212) angeordnet ist, zum selektiven Führen eines Abschnitts des Steuerelements (264) zwischen dem ersten und dem zweiten Bereich (212a, 212b), wodurch ein Biegemoment, das auf den distalen Abschnitt (215) aufgebracht wird, wenn eine axiale Kraft auf ein proximales Ende des Steuerelements (264) aufgebracht wird, geändert wird.

**14.** Vorrichtung gemäß Anspruch 13, wobei das Steuer-Anpassungs-Element (280) ein Querschnittsprofil aufweist, das größer als der zweite Bereich (212b) des Durchgangs (212) ist, wodurch verhindert wird, dass das Steuer-Anpassungs-Element (280) in den zweiten Bereich (212b) des Durchgangs (212) eintritt.

**15.** Vorrichtung gemäß Anspruch 13, wobei das Steuerelement (264) in einem Lumen (286) angeordnet ist, das in dem Steuer-Anpassungs-Element (280) ausgebildet ist.

**16.** Vorrichtung gemäß Anspruch 13, wobei der Durchgang (212) einen schlüssellochförmigen Querschnitt aufweist.

**17.** Vorrichtung gemäß Anspruch 1, wobei:

der Durchgang (420) des röhrenförmigen Elements (412) einen länglichen geschlitzten Bereich (420c) aufweist, der den ersten und den zweiten Bereich (420a, 420b) kreuzt,
ein proximaler Abschnitt des Steuerelements (464) in dem ersten Bereich (420a) des Durchgangs (420) angeordnet ist, ein distaler Abschnitt des Steuerelements (464) in dem zweiten Bereich (420b) des Durchgangs (420) angeordnet ist, und das distale Ende im zweiten Bereich (420b) an dem röhrenförmigen Element (412) befestigt ist, und

das Steuer-Anpassungs-Element (480) gleitend in dem länglichen geschlitzten Bereich (420c) des Durchgangs (420) angeordnet ist, zum selektiven Führen eines Abschnitts des Steuerelements (464) zwischen dem ersten und dem zweiten Bereich (420a, 420b), wodurch ein Biegemoment, das auf den distalen Abschnitt aufgebracht wird, wenn eine axiale Kraft auf ein proximales Ende des Steuerelements (464) aufgebracht wird, geändert wird.

18. Vorrichtung gemäß Anspruch 17, wobei das Steuer-Anpassungs-Element (480) einen im Wesentlichen flachen Körper aufweist, der gleitend in dem geschlitzten Bereich (420c) aufgenommen ist.

19. Vorrichtung gemäß einem vorhergehenden Anspruch, ferner eine optische Bildgebungsanordnung (60) aufweisend, die zum bildlichen Darstellen der Gewebestruktur über das distale Ende hinaus von dem distalen Ende des röhrenförmigen Elements getragen wird, wobei das Steuerelement einen Lichtleiter (62, 64) aufweist, der mit der Bildgebungsanordnung (60) verbunden ist.

20. Vorrichtung gemäß Anspruch 19, ferner ein im Wesentlichen transparentes dehnbares Element (50) aufweisend, das von dem distalen Ende des röhrenförmigen Elements getragen wird, wobei das dehnbare Element (50) von einem zusammengelegten Zustand in einen ausgedehnten Zustand dehnbar ist, und zumindest teilweise die Bildgebungsanordnung (60) zum bildlichen Darstellen über das distale Ende hinaus durch das expandierbare Element (50) hindurch umgibt.

21. Vorrichtung gemäß einem vorhergehenden Anspruch, wobei das Steuerelement (164) eine optische Bilddarstellungsfaser (64) aufweist.

22. Vorrichtung gemäß einem der Ansprüche 1 bis 20, wobei das Steuerelement (164) eine Beleuchtungsfaser (62) aufweist.

## Revendications

1. Appareil pour introduction dans une lumière corporelle, comprenant un élément tubulaire (112, 212, 412), comprenant une extrémité proximale, une extrémité distale (116, 216) calibrée pour introduction dans une lumière corporelle, et un passage (120, 220, 420) s'étendant le long d'une portion distale orientable (115, 215) de l'élément tubulaire, le passage (120, 220, 420) comprenant une première région (120a, 220a, 420a) et une seconde région (120b, 220b, 420b) décalée d'un centre de module (184, 284, 484) de la portion distale (115, 215) ; et un élément de direction (164, 264, 464) disposé de manière à coulisser à travers le passage (120, 220, 420) s'étendant le long de la portion distale (115, 215), l'élément de direction (164, 264, 464) comprenant une extrémité proximale disposée de manière adjacente par rapport à l'extrémité proximale de l'élément tubulaire, et une extrémité distale couplée à l'extrémité distale (116) de l'élément tubulaire, au-delà de la portion distale (115, 215), **caractérisé en ce que :**

la première région (120a, 220a, 420a) est sensiblement alignée avec le centre de module (184, 284, 484) de la portion distale (115, 215) ; et
un élément d'ajustement de direction (180, 280, 480) disposé de manière à coulisser dans le passage (120, 220 420) pour diriger de manière sélective une portion de l'élément de direction (164, 264 464) entre les première et seconde régions (120a, 220a, 420a, 120b, 220b, 420b), modifiant ainsi un moment de flexion appliqué à la portion distale (115, 215) lorsqu'une force axiale est appliquée à l'extrémité proximale de l'élément de direction (164, 264, 464).

2. Appareil selon la revendication 1, dans lequel l'élément d'ajustement de direction comprend un guide (180, 280) pouvant coulisser dans une des première et seconde régions (120a, 220a, 120b, 220b) pour contraindre une première portion de l'élément de direction (164, 264) à l'intérieur de la première région (120a, 220a) proximale à une pointe distale (182, 282) du guide (180, 280), tout en permettant à une seconde portion de l'élément de direction (164, 264) distale à la pointe distale (182, 282) d'entrer dans la seconde région (120b, 220b).

3. Appareil selon la revendication 2, dans lequel la seconde région (220b) a une coupe transversale plus petite que la première région (220a), le guide (280) ayant une coupe transversale plus grande que la seconde région (220b) qui peut coulisser de manière axiale dans la première région (220a).

4. Appareil selon la revendication 3, dans lequel le guide (480') comprend une coupe transversale arquée définissant un évidement (486') pour y recevoir une portion de l'élément de direction (464').

5. Appareil selon la revendication 2, dans lequel le guide (180) peut coulisser axialement dans la seconde région (120b), empêchant ainsi l'élément de direction (164) d'entrer dans la seconde région (120b) proximale à une pointe distale (182) du guide.

**6.** Appareil selon la revendication 2, dans lequel le guide (280) peut coulisser axialement dans la première région (220a).

**7.** Appareil selon la revendication 1, dans lequel le passage (120, 220) comprend une coupe transversale en trou de serrure définissant les première et seconde régions (120a, 220a, 120b, 220b).

**8.** Appareil selon la revendication 1, dans lequel :

l'élément de direction (164) est disposé de manière à coulisser à travers le passage (120), une portion proximale de l'élément de direction (164) étant disposée dans la première région (120a) du passage (120) et une portion distale de l'élément de direction (164) étant disposée dans la seconde région (120b) du passage (120), et l'extrémité distale est couplée à l'élément tubulaire (112) dans la seconde région (120b) ; et l'élément d'ajustement de direction (180) est disposé de manière à coulisser dans la seconde région (120b) du passage (120) pour diriger de manière sélective une portion de l'élément de direction (164) entre les première et seconde régions (120a, 120b), modifiant ainsi un moment de flexion appliqué à la portion distale (115) lorsqu'une force axiale est appliquée à une extrémité proximale de l'élément de direction (164).

**9.** Appareil selon la revendication 8, dans lequel le passage (120) a une coupe transversale en forme de trou de serrure.

**10.** Appareil selon la revendication 8, dans lequel l'élément de direction (164) a une coupe transversale plus petite que les première et seconde régions (120a, 120b).

**11.** Appareil selon la revendication 8, dans lequel les première et seconde régions (120a, 120b) se chevauchent, créant ainsi une nervure ou une division (120c) entre les première et seconde régions (120a, 120b).

**12.** Appareil selon la revendication 11, dans lequel l'élément d'ajustement de direction (164) a une coupe transversale qui est plus petite que la seconde région (120b), mais plus grande qu'une largeur de la division (120c).

**13.** Appareil selon la revendication 1, dans lequel :

l'élément de direction (264) est disposé de manière à coulisser à travers le passage (220), une portion proximale de l'élément de direction (264) étant disposée dans la première région (220a) du passage (220) et une portion distale de l'élément de direction (264) étant disposée dans la seconde région (220b) du passage (220), et l'extrémité distale est fixée à l'élément tubulaire (212) dans la seconde région (220b) ; et l'élément d'ajustement de direction (280) est disposé à coulissement dans la première région (212a) du passage (212) pour diriger sélectivement une portion de l'élément de direction (264) entre les première et seconde régions (212a, 212b), modifiant ainsi un moment de flexion appliqué à la portion distale (215) lorsqu'une force axiale est appliquée à une extrémité proximale de l'élément de direction (264).

**14.** Appareil selon la revendication 13, dans lequel l'élément d'ajustement de direction (280) a un profil en coupe transversale qui est plus grand que la seconde région (212b) du passage (212), empêchant ainsi l'élément d'ajustement de direction (280) d'entrer dans la seconde région (212b) du passage (212).

**15.** Appareil selon la revendication 13, dans lequel l'élément de direction (264) est disposé dans une lumière (286) formée dans l'élément d'ajustement de direction (280).

**16.** Appareil selon la revendication 13, dans lequel le passage (212) a une coupe transversale en forme de trou de serrure.

**17.** Appareil selon la revendication 1, dans lequel :

le passage (420) de l'élément tubulaire (412) comprend une région allongée fendue (420c) coupant les première et seconde régions (420a, 420b) ; une portion proximale de l'élément de direction (464) est disposée dans la première région (420a) du passage (420), une portion distale de l'élément de direction (464) est disposée dans la seconde région (420b) du passage (420), et l'extrémité distale est fixée à l'élément tubulaire (412) dans la seconde région (420b) ; et l'élément d'ajustement de direction (480) est disposé de manière à coulisser dans la région allongée fendue (420c) du passage (420) pour diriger sélectivement une portion de l'élément de direction (464) entre les première et seconde régions (420a, 420b), modifiant ainsi un moment de flexion appliqué à la portion distale lorsqu'une force axiale est appliquée à une extrémité proximale de l'élément de direction (464).

**18.** Appareil selon la revendication 17, dans lequel l'élément d'ajustement de direction (480) comprend un corps sensiblement plat qui est reçu de manière à coulisser dans la région fendue (420c).

**19.** Appareil selon l'une quelconque des revendications précédentes, comprenant en outre un ensemble d'imagerie optique (60) porté par l'extrémité distale de l'élément tubulaire pour former l'image de la structure tissulaire au-delà de l'extrémité distale, dans lequel l'élément de direction comprend une fibre optique (62, 64) couplée à l'ensemble d'imagerie (60).

**20.** Appareil selon la revendication 19, comprenant en outre un élément extensible sensiblement transparent (50) porté par l'extrémité distale de l'élément tubulaire, l'élément extensible (50) pouvant passer d'un état affaissé à un état déployé et entourant au moins en partie l'ensemble d'imagerie (60) pour former une image au-delà de l'extrémité distale à travers l'élément extensible (50).

**21.** Appareil selon l'une quelconque des revendications précédentes, dans lequel l'élément de direction (164) comprend une fibre d'imagerie optique (64).

**22.** Appareil selon l'une quelconque des revendications 1 à 20, dans lequel l'élément de direction (164) comprend une fibre d'éclairage (62).

*FIG. 1*

*FIG. 2*

FIG. 3

FIG. 4A

FIG. 4B

FIG. 4C

FIG. 4D

FIG. 4E

FIG. 5

*FIG. 6A*

36

*FIG. 6B*

*FIG. 7*

FIG. 8A

FIG. 8B

FIG. 9A

FIG. 9B

FIG. 9C

FIG. 10A

FIG. 10B

FIG. 10C

FIG. 10D

FIG. 11A

FIG. 11B

FIG. 12A

FIG. 12B

FIG. 13A

FIG. 13B

FIG. 14

FIG. 15

FIG. 16A

FIG. 16B

FIG. 17

FIG. 18

FIG. 19

FIG. 20

FIG. 21A

FIG. 21B

FIG. 23

FIG. 22

312a

380a

364a

FIG. 24A

380b

386b

FIG. 24B

380c

386c

FIG. 24C

380d

386d

FIG. 24D

380e

386e

FIG. 24E

380f

386f

FIG. 24F

412

420b

420c

420a

484

FIG. 25A

412

420b

480

420c

420a

464

FIG. 25B

412'

480'

420b'

486'

464'

420c'

484'

FIG. 26

512'

520b

580

520c

520a

584

564

L

FIG. 27

FIG. 28

FIG. 29

FIG. 30A

FIG. 30B

FIG. 31A

FIG. 31B

EP 1 727 459 B1

FIG. 32A

FIG. 32B

FIG. 33A

FIG. 33B

FIG. 33C

**FIG. 34**

**FIG. 35**

**FIG. 36**

FIG. 37

FIG. 38

FIG. 39

FIG. 40

FIG. 41

FIG. 42

FIG. 43

FIG. 44

*FIG. 45*

1015a

1016

1015

1015b

1013

1012

**FIG. 46A**

47

1015a

1016

1015

47

1015b

1013

1012

**FIG. 46B**

1020c

1020d

1015a

1020b

1020a

1020d

1015b

1020b

**FIG. 47**

1116    49A    1115    1110    1112

1130    49A    1164

FIG. 48A

49B

1116    1115    1110    1112

49B

1130    1164

FIG. 48B

1110

1110    1112

1112

1164

1130    1164

1130

FIG. 49A    FIG. 49B

FIG. 50A

FIG. 50B

FIG. 51A

FIG. 51B

FIG. 52

FIG. 53A

FIG. 53B

*50*

*1456*

**FIG. 53C**

LENS

∅

Z

X

Fov=2Z=Zxtan∅

**FIG. 53D**

*50*

*1502*

*1504*

**FIG. 54**

*50*

*1506*

**FIG. 55**

*FIG. 56*

*FIG. 57*

*FIG. 58*

FIG. 59

FIG. 60

FIG. 61

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5489270 A
- US 10447526 B
- US 6283951 B
- US 10433321 B
- US 10934082 B
- US 10958035 B